# EUROPÄISCHE PATENTSCHRIFT

(11) **EP 1 694 728 B1**
(45) Veröffentlichungstag und Bekanntmachung des Hinweises auf die Patenterteilung: **13.08.2008**
(21) Anmeldenummer: 04803650.3
(22) Anmeldetag: 08.12.2004
(51) Int. Cl.: A61Q 5/06, C08F 220/54

(54) **COPOLYMERE AUF BASIS VON TERT.-BUTYL(METH)ACRYLAT UND DEREN VERWENDUNG IN HAARSPRAYS**
COPOLYMERS BASED ON TERT-BUTYL(METH)ACRYLATE AND USE THEREOF IN HAIRSPRAYS
COPOLYMERES A BASE DE TERT.-BUTYL(METH)ACRYLATE ET LEUR UTILISATION DANS LAQUES A CHEVEUX

(30) Priorität: 09.12.2003 DE 10357486
(43) Veröffentlichungstag der Anmeldung: 30.08.2006
(73) Patentinhaber: BASF SE, 67056 Ludwigshafen (DE)
(72) Erfinder: NGUYEN-KIM, Son, 69502 Hemsbach (DE); WOOD, Claudia, 69469 Weinheim (DE)
(74) Vertreter: Reitstötter - Kinzebach
(86) Internationale Anmeldenummer: PCT/EP2004/013984
(87) Internationale Veröffentlichungsnummer: WO 2005/058989

(56) Entgegenhaltungen:
- DE-A1- 4 314 305
- US-A- 3 632 838
- US-A- 3 927 199
- US-A- 4 748 989
- US-A- 5 025 062

## Beschreibung

Die vorliegende Erfindung betrifft Copolymere, die tert.-Butyl(meth)acrylat, wenigstens eine a,β-ethylenisch ungesättigte amidgruppenhaltige Verbindung und Acrylsäure einpolymerisiert enthalten, kosmetische und pharmazeutische Mittel, die wenigstens ein solches Copolymer enthalten sowie die Verwendung dieser Copolymere.

Polymere mit filmbildenden Eigenschaften haben vielfältige Anwendungen im Bereich der Pharmazie und Kosmetik gefunden. In der Pharmazie dienen sie beispielsweise als Beschichtungsmittel oder Bindemittel für feste Arzneiformen. In der Kosmetik werden Polymere mit filmbildenden Eigenschaften u. a. zur Festigung, Strukturverbesserung und Formgebung der Haare verwendet. Sie dienen beispielsweise als Conditioner dazu, die Trocken- und Nasskämmbarkeit, das Anfassgefühl, den Glanz und die Erscheinungsform zu verbessern sowie dem Haar antistatische Eigenschaften zu verleihen. Anforderungen, die an filmbildende Polymere für einen Einsatz als Festigerharze gestellt werden, sind z. B. eine starke Festigung (auch bei hoher Luftfeuchtigkeit), Elastizität, Auswaschbarkeit vom Haar, Verträglichkeit in der Formulierung und ein angenehmer Griff des damit behandelten Haares. Schwierigkeiten bereitet oft die Bereitstellung von Produkten mit einem komplexen Eigenschaftsprofil. So besteht ein Bedarf an filmbildenden Polymeren für haarkosmetischen Mittel, die zur Bildung im Wesentlichen glatter, klebfreier Filme befähigt sind, eine gute Festigungswirkung aufweisen und dem Haar gleichzeitig gute sensorisch erfassbare Eigenschaften, wie Elastizität und einen angenehmen Griff, verleihen. Sollen diese Polymere in Haarsprayformulierungen eingesetzt werden, so ist zudem eine gute Treibgasverträglichkeit, die Eignung für einen Einsatz in Low-VOC-Formulierungen (VOC = Volatile Organic Compounds), eine gute Versprühbarkeit, eine gute Löslichkeit in Wasser oder wässrig/alkoholischen Lösungsmittelgemischen und eine gute Auswaschbarkeit erwünscht.

Es ist bekannt, Polymere auf Basis von tert.-Butyl(meth)acrylat in Haarkosmetika einzusetzen. So beschreibt die DE-A-32 27 334 (EP-A-0 100 890) Copolymerisate, die durch radikalische Copolymerisation von a) mindestens einem C₂-C₂₀-(Meth)acrylsäurealkylester, z. B. tert.-Butyl(meth)acrylat, b) mindestens einem wasserlöslichen stickstoffhaltigen Monomer, z. B. einem N-Vinyllactam, c) wenigstens einem Monomer mit kationischen Gruppen und d) wenigstens einer radikalisch polymerisierbaren Carbonsäure erhältlich sind.

Die DE-A-36 27 970 und die DE-A-40 31 912 beschreiben Terpolymere aus Vinylpyrrolidon, tert.-Butyl(meth)acrylat und Acrylsäure oder Methacrylsäure und deren Verwendung in Haarbehandlungsmitteln.

Die DE-A-43 14 305 beschreibt ein Haarfestigungsmittel, das als Filmbildner ein Copolymerisat auf Basis von tert.-Butylacrylat oder tert.-Butyl(meth)acrylat, Acrylsäure oder Methacrylsäure sowie einem radikalisch copolymerisierbaren Monomeren, welches ein Homopolymerisat mit einer Glastemperatur < 30 °C liefert, enthält.

Die DE-A-100 08 263 beschreibt ein kosmetisches Mittel, das wenigstens ein wasserlösliches oder wasserdispergierbares Polymer enthält, welches höchstens 50 Gew.-% wenigstens eines tert.-Butylesters und/oder N-tert.-Butylamids einer a,β-ethylenisch ungesättigten Carbonsäure sowie wenigstens ein N-Vinylamid und/oder Vinyllactam und wenigstens eine polymerisierbare Verbindung mit einer kationogenen und/oder kationischen Gruppe einpolymerisiert enthält.

Die WO 01/62809 beschreibt ein kosmetisches Mittel, das wenigstens ein wasserlösliches oder wasserdispergierbares Polymer enthält, das
a) 5 bis 50 Gew.-% wenigstens eines α,β-ethylenisch ungesättigten Monomers mit einer tert.-Butylgruppe,
b) 25 bis 90 Gew.-% wenigstens eines N-Vinylamids und/oder N-Vinyllactams,
c) 0,5 bis 30 Gew.-% wenigstens einer Verbindung mit einer radikalisch polymerisierbaren, α,β-ethylenisch ungesättigten Doppelbindung und mindestens einer kationogenen und/oder kationischen Gruppe pro Molekül, und
d) 0 bis 30 Gew.-% wenigstens einer weiteren α,β-ethylenisch ungesättigten Verbindung, wobei es sich um Verbindungen mit mindestens einer anionogenen und/oder anionischen Gruppe pro Molekül handeln kann,
eingebaut enthält.

Es ist bekannt, Copolymere auf Basis von N-Alkyl(meth)acrylamiden in kosmetischen Mitteln einzusetzen. Die US 3,927,199 beschreibt eine Haarfestigerzusammensetzung, die ein filmbildendes Binderharz auf Basis eines Copolymers enthält, das 1) N-Alkylacrylamide oder Methacrylamide, 2) säuregruppenhaltige Monomere und 3) wenigstens ein weiteres Comonomer einpolymerisiert enthält.

Die EP-A-0 062 002 beschreibt eine Haarfixierungszubereitung, die ein Terpolymer aus einem N-Alkyl(meth)acrylamid, einen C₁-C₄-Alkylester oder C₁-C₄-Hydroxyalkylester der (Meth)acrylsäure und Acrylsäure der Methacrylsäure enthalten. Terpolymere auf Basis von tert.-Butyl(meth)acrylat sind nicht offenbart.

Die unveröffentlichte deutsche Patentanmeldung P 102 61 750.3 beschreibt ein ampholytisches Copolymer, das durch radikalische Copolymerisation von
a) wenigstens einer ethylenisch ungesättigten Verbindung mit mindestens einer anionogenen und/oder anionischen Gruppe,
b) wenigstens einer ethylenisch ungesättigten Verbindung mit mindestens einer kationogenen und/oder kationischen Gruppe,
c) wenigstens einer ungesättigten amidgruppenhaltigen Verbindung
   sowie gegebenenfalls weiterer Comonomere erhältlich sind. Beschrieben sind weiterhin Polyelektrolyt-Komplexe, die ein solches ampholytisches Copolymer enthalten sowie kosmetisch oder pharmazeutische Mittel auf Basis dieser ampholytischen Copolymere und Polyelektrolyt-Komplexe.

Die unveröffentlichte deutsche Patentanmeldung 102 37 378.7 beschreibt die Verwendung von Polymeren, die erhältlich sind durch
(i) radikalisch initiierte Copolymerisation von Monomergemischen aus
   (a) mindestens einem kationischen Monomeren oder quaternisierbaren Monomeren,
   (b) gegebenenfalls einem wasserlöslichen Monomeren,
   (c) gegebenenfalls einem weiteren radikalisch copolymerisierbaren Monomeren,
   (d) mindestens einem als Vernetzer wirkenden Monomeren mit mindestens zwei ethylenisch ungesättigten, nichtkonjugierten Doppelbindungen, und
   (e) mindestens einem Regler,
(ii) anschließende Quaternisierung oder Protonierung des Polymeren, sofern als Monomeres (a) ein nicht oder nur partiell quaternisiertes Monomer eingesetzt wird,
in haarkosmetischen Zubereitungen.

Die unveröffentlichte deutsche Patentanmeldung 103 31 865.8 beschreibt eine wässrige Polymerdispersion Pd), die erhältlich ist durch radikalische Polymerisation eines Monomergemischs M), enthaltend
a) wenigstens eine α,β-ethylenisch ungesättigte amidgruppenhaltige Verbindung der allgemeinen Formel I 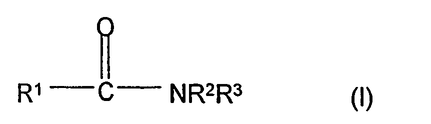 wobei
   R² für eine Gruppe der Formel CH₂=CR⁴- steht und R¹ und R³ unabhängig voneinander für H, Alkyl, Cycloalkyl, Heterocycloalkyl, Aryl oder Hetaryl stehen, oder
   R¹ und R³ gemeinsam mit der Amidgruppe, an die sie gebunden sind, für ein Lactam mit 5 bis 8 Ringatomen stehen,
b) wenigstens eine radikalisch polymerisierbare vernetzende Verbindung mit wenigstens zwei α,β-ethylenisch ungesättigten Doppelbindungen pro Molekül,
c) wenigstens eine Verbindung mit einer radikalisch polymerisierbaren α,β-ethylenisch ungesättigten Doppelbindung und mindestens einer kationogenen und/oder kationischen Gruppe pro Molekül,
in einem wässrigen Medium in Gegenwart wenigstens eines polymeren anionischen Dispergiermittels D), Sie eignen sich als Konditioniermittel für kosmetische Zubereitungen, insbesondere Shampoos.

Die unveröffentlichte deutsche Patentanmeldung 102 37 378.7 beschreibt ein kosmetisches oder pharmazeutisches Mittel, das wenigstens einen Polyelektrolyt-Komplex umfasst, der als Komponente A1) wenigstens ein wasserlösliches oder wasserdispergierbares Copolymer mit kationogenen Gruppen, das Vinylimidazol und/oder ein Derivat davon und wenigstens ein weiteres damit copolymerisierbares Monomer einpolymerisiert enthält, und als Komponente A2) wenigstens ein säuregruppenhaltiges Polymer enthält.

Die US 5,025,062 beschreibt Beschichtungsmittel auf Basis von synthetischen Harzen, die wenigstens einen tert.-Butylester einer α,β-ethylenisch ungesättigten Carbonsäure oder Dicarbonsäure einpolymerisiert enthalten.

Die US 3,632,838 beschreibt Oberflächenbeschichtungen auf Basis von Copolymeren, die veretherte N-Methylolamidgruppen enthalten. Die Copolymere können tert.-Butylacrylat einpolymerisiert enthalten.

Die US 3,927,199 beschreibt eine Haarfestigerzusammensetzung, die ein Copolymer enthält, welches (1) wenigstens ein N-Alkyl(meth)acrylamid, (2) wenigstens ein säuregruppenhaltiges Monomer, das ausgewählt ist unter Acrylsäure, Methacrylsäure, Crotonsäure, Itaconsäure, Maleinsäure, Fumarsäure und den C₁-C₄-Alkylhalbestern der Malein- und Fumarsäure und (3) wenigstens ein damit copolymerisierbares Comonomer einpolymerisiert enthält.

Die US 4,748,989 beschreibt ein Copolymer für Haarfestiger, das (A) Vinylpyrrolidon, (B) wenigstens ein mono- oder dialkyliertes Acrylamid und (C) wenigstens ein weiteres Monomer, das ausgewählt ist unter Alkyl- und Hydroxyalkyl(meth)acrylaten, Acrylsäure oder Methacrylsäure einpolymerisiert enthält.

Trotz umfangreicher Bemühungen besteht nach wie vor Verbesserungsbedarf bei den aus dem Stand der Technik bekannten Polymeren zur Erzeugung von Haarsprayformulierungen mit einem geringen VOC-Wert, guten rheologischen Eigenschaften und einer guten Sprühbarkeit sowie bei gleichzeitig starker Festigung (auch bei hoher Luftfeuchtigkeit). Überraschenderweise wurde ein Haarspray gefunden, enthaltend
A) wenigstens ein Copolymer, erhältlich durch radikalische Polymerisation eines Monomergemischs, enthaltend
   a) tert.-Butylacrylat und/oder tert.-Butylmethacrylat, wobei die Komponente a) tert.-Butylacrylat umfasst oder daraus besteht,
   b) wenigstens eine α,β-ethylenisch ungesättigte amidgruppenhaltige Verbindung der allgemeinen Formel I 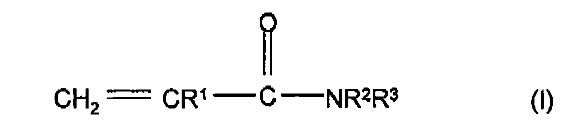 worin
      R¹ für H oder C₁-C₄-Alkyl steht,
      R² und R³ unabhängig voneinander für H oder C₁-C₄-Alkyl stehen oder R² und R³ gemeinsam mit dem Stickstoffatom, an das sie gebunden sind, auch für einen 4- bis 7-gliedrigen Heterocyclus stehen können,
      mit der Maßgabe, dass die Summe der Kohlenstoffatome der Reste R¹, R² und R³ höchstens 4 beträgt, und
   c) Acrylsäure, und
B) wenigstens einen kosmetisch oder pharmazeutisch akzeptablen Träger.

Im Folgenden werden Verbindungen, die sich von Acrylsäure und Methacrylsäure ableiten können, teilweise verkürzt durch Einfügen der Silbe "(meth)" in die von der Acrylsäure abgeleitete Verbindung bezeichnet.

Unter wasserlöslichen Monomeren und Polymeren werden im Rahmen der vorliegenden Erfindung Monomere und Polymere verstanden, die sich zu mindestens 1 g/l bei 20 °C in Wasser lösen. Unter wasserdispergierbaren Monomeren und Polymeren werden Monomere und Polymere verstanden, die unter Anwendung von Scherkräften, beispielsweise durch Rühren, in dispergierbare Partikel zerfallen. Hydrophile Monomere sind vorzugsweise wasserlöslich oder zumindest wasserdispergierbar. Die erfindungsgemäßen Copolymere sind im Allgemeinen wasserlöslich.

Die erfindungsgemäßen Copolymere eignen sich in besonders vorteilhafter Weise für einen Einsatz in kosmetischen Mitteln, insbesondere in Haarbehandlungsmittein. Sie dienen bevorzugt zur Erzeugung elastischer Frisuren bei gleichzeitig starker Festigung. Vorteilhafterweise zeichnen sie sich zudem sowohl durch eine gute Treibgasverträglichkeit, als auch eine gute Löslichkeit in Wasser oder wässrig/alkoholischen Lösungsmittelgemischen aus. Sie lassen sich somit sowohl zu hoch treibgashaltigen Haarsprays (VOC mindestens 85 Gew.-%) als auch zu Formulierungen mit geringen VOC-Werten (im Allgemeinen nicht mehr als 55 Gew.%, bezogen auf das Gesamtgewicht des Mittels) formulieren. Dabei zeichnen sich die Haarsprayformulierungen in jedem Fall durch eine sehr gute Sprühbarkeit aus.

Zur Einstellung bestimmter Produkteigenschaften kann ein Teil der Monomere a) durch wenigstens ein Monomer f) ersetzt sein. In einer speziellen Ausführung können daher die Monomere der Komponente a) zu bis zu 50 Ges.-% durch wenigstens ein C₁-C₃-Alkylmethacrylat und/oder Hydroxy-C₁-C₃-alkylmethacrylat ersetzt sein. Geeignete C₁-C₃-Alkylmethacrylate und Hydroxy-G₁-C₃-alkylmethacrylate sind im Folgenden bei der Komponente f) beschrieben. Bevorzugt wird Ethylmethacrylat eingesetzt.

### Monomer a)

Die erfindungsgemäßen Copolymere enthalten als Komponente a) tert.-Butylacrylat und/oder tert.-Butylmethacrylat einpolymerisiert. Vorzugsweise wird tert.-Butylmethacrylat nicht alleine als Komponente a) eingesetzt. Bevorzugt sind daher Copolymere, wobei die Komponente a) tert.-Butylacrylat umfasst oder daraus besteht.

Die erfindungsgemäßen Copolymere enthalten vorzugsweise 30 bis 90 Gew.-%, besonders bevorzugt 40 bis 85 Gew.-%, bezogen auf das Gesamtgewicht der zur Polymerisation eingesetzten Monomere, wenigstens einer Verbindung a) einpolymerisiert.

### Monomer b)

Als Komponente b) enthalten die Copolymere wenigstens ein Amid einer α,β-ethylenisch ungesättigten Monocarbonsäure der allgemeinen Formel I einpolymerisiert. Bevorzugt stehen in den Verbindungen der allgemeinen Formel I die Reste R² und R³ beide für H oder steht einer der Reste R² und R³ für H und der andere für C₁-C₄-Alkyl.

Vorzugsweise leiten sich die Amide der allgemeinen Formel I von Acrylsäure, Methacrylsäure oder Ethacrylsäure als α,β-ethylenisch ungesättigter Monocarbonsäure ab. Vorzugsweise ist die Komponente b) ausgewählt unter Acrylsäureamid, Methacrylsäureamid, N-Methyl(meth)acrylamid, N-Ethyl(meth)acrylamid,
N-Propyl(meth)acrylamid, N-(n-Butyl)acrylamid, N-(sek.-Butyl)acrylamid,
N-(tert.-Butyl)acrylamid, N,N-Dimethyl(meth)acrylamid,
N,N-Diethylacrylamid, N-Acryloylmorpholin, N-Acryloylpiperazin,
N-(Meth)acryloylpyrrolidin und Mischungen davon.

Bevorzugt umfasst die Komponente b) Methacrylsäureamid und/oder N-(tert.-Butyl)acrylamid oder besteht aus einer dieser Komponenten oder aus einem Gemisch von Methacrylsäureamid und N-(tert.-Butyl)acrylamid.

Die erfindungsgemäßen Copolymere enthalten vorzugsweise 3 bis 50 Gew.-%, besonders bevorzugt 5 bis 40 Gew.-%, insbesondere 10 bis 35 Gew.-%, bezogen auf das Gesamtgewicht der zur Polymerisation eingesetzten Monomere, wenigstens einer Verbindung der Komponente b) einpolymerisiert.

### Monomer c)

Die erfindungsgemäßen Copolymere enthalten vorzugsweise 5 bis 40 Gew.-%, besonders bevorzugt 7 bis 35 Gew.-%, insbesondere 10 bis 30 Gew.-%, bezogen auf das Gesamtgewicht der zur Polymerisation eingesetzten Monomere, Acrylsäure (= Monomer c)) einpolymerisiert.

Zur Herstellung der Copolymere kann die Acrylsäure c) in teilweise oder vollständig deprotonierter Form eingesetzt werden. Dann leiten sich deren Gegenionen vorzugsweise von Basen ab, wie sie im Folgenden zur Einstellung des pH-Werts bei der Polymerisation oder der erhaltenen Polymerisate beschrieben werden.

In einer speziellen Ausführungsform bestehen die erfindungsgemäßen Copolymere nur aus Monomereinheiten der zuvor genannten Monomere a), b) und c). In weiteren Ausführungsformen enthalten die erfindungsgemäßen Copolymere zusätzliche Monomere einpolymerisiert. Geeignete zusätzliche Monomere werden im Folgenden aufgeführt.

### Monomer d)

In einer bevorzugten Ausführungsform enthält das Copolymer zusätzlich Methacrylsäure (= Monomer d)) einpolymerisiert. Bevorzugt enthalten die Copolymere dann bis zu 25 Gew.-%, besonders bevorzugt bis zu 20 Gew.-%, insbesondere bis zu 15 Gew.-%, bezogen auf das Gesamtgewicht der zur Polymerisation eingesetzten Monomere Methacrylsäure einpolymerisiert. Wenn Methacrylsäure zur Polymerisation eingesetzt wird, dann vorzugsweise in einer Menge von mindestens 1 Gew.-%, besonders bevorzugt mindestens 5 Gew.-%.

### Monomer e)

Die erfindungsgemäßen Copolymere können zusätzlich wenigstens eine Verbindung mit einer radikalisch polymerisierbaren, α,β-ethylenisch ungesättigten Doppelbindung und wenigstens einer kationogenen und/oder kationischen Gruppe pro Molekül einpolymerisiert enthalten.

Bevorzugt handelt es sich bei den kationogenen bzw. kationischen Gruppen der Komponente e) um stickstoffhaltige Gruppen, wie primäre, sekundäre und tertiäre Aminogruppen sowie quaternäre Ammoniumgruppen. Vorzugsweise handelt es sich bei den stickstoffhaltigen Gruppen um tertiäre Aminogruppen. Bevorzugt werden die Verbindungen e) in nicht geladener Form zur Polymerisation eingesetzt. Geeignet ist jedoch auch ein Einsatz in geladener Form. Geladene kationische Gruppen lassen sich z. B. aus den Aminstickstoffen durch Protonierung, z. B. mit einwertigen oder mehrwertigen Carbonsäuren, wie Milchsäure oder Weinsäure, oder Mineralsäuren, wie Phosphorsäure, Schwefelsäure und Salzsäure erzeugen.

Vorzugsweise ist die Komponente e) ausgewählt ist unter Estern α,β-ethylenisch ungesättigter Mono- und Dicarbonsäuren mit Aminoalkoholen, welche am Aminstickstoff mono- oder dialkyliert sein können, Amiden α,β-ethylenisch ungesättigter Mono- und Dicarbonsäuren mit Diaminen, welche mindestens eine primäre oder sekundäre Aminogruppe aufweisen, N,N-Diallylamin, N,N-Diallyl-N-alkylaminen und deren Derivaten, vinyl- und allylsubstituierten Stickstoffheterocyclen, vinyl- und allylsubstituierten heteroaromatischen Verbindungen und Mischungen davon.

Geeignet als Verbindungen e) sind die Ester von α,β-ethylenisch ungesättigten Mono- und Dicarbonsäuren mit Aminoalkoholen. Bevorzugte Aminoalkohole sind C₂-C₁₂-Aminoalkohole, welche am Aminstickstoff C₁-C₈-mono- oder -dialkyliert sind. Als Säurekomponente dieser Ester eignen sich z. B. Acrylsäure, Methacrylsäure, Fumarsäure, Maleinsäure, Itaconsäure, Crotonsäure, Maleinsäureanhydrid, Monobutylmaleat und Gemische davon. Bevorzugt werden Acrylsäure, Methacrylsäure und deren Gemische eingesetzt. Besonders bevorzugt als Verbindungen c2) sind N-Methylaminoethyl(meth)acrylat, N-Ethylaminoethyl(meth)acrylat, N-(n-Propyl)aminoethyl(meth)acrylat, N-(n-Butyl)aminoethyl(meth)acrylat, N-(tert.-Butyl)aminoethyl(meth)acrylat, N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat und N,N-Dimethylaminocyclohexyl(meth)acrylat. Insbesondere werden als Verbindung e) N-(tert.-Butyl)aminoethylacrylat und N-(tert.-Butyl)aminoethylmethacrylat eingesetzt.

Geeignete Monomere e) sind weiterhin die Amide der zuvor genannten α,β-ethylenisch ungesättigten Mono- und Dicarbonsäuren mit Diaminen, welche mindestens eine primäre oder sekundäre Aminogruppe aufweisen. Bevorzugt sind Diamine, die eine tertiäre und eine primäre oder sekundäre Aminogruppe aufweisen. Bevorzugt werden als Monomere e) N-[2-(dimethylamino)ethyl]acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)-butyl]methacrylamid, N-[2-(diethylamino)ethyl]acrylamid, N-[4-(dimethylamino)cyclohexyl]acrylamid und N-[4-(dimethylamino)cyclohexyl]-methacrylamid eingesetzt. Besonders bevorzugt werden N-[3-(dimethyl-amino)propyl]acrylamid und/oder N-[3-(dimethylamino)propyl]methacrylamid eingesetzt.

Geeignete Monomere e) sind weiterhin N,N-Diallylamine und N,N-Diallyl-N-alkylamine und deren Säureadditionssalze. Alkyl steht dabei vorzugsweise für C₁-C₂₄-Alkyl. Bevorzugt ist z. B. N,N-Diallyl-N-methylamin.

Geeignete Monomere e) sind weiterhin vinyl- und allylsubstituierte Stickstoffheterocyclen, wie N-Vinylimidazol, N-Vinylimidazol-Derivate, z. B. N-Vinyl-2-methylimidazol, vinyl- und allylsubstituierte heteroaromatische Verbindungen, wie 2- und 4-Vinylpyridin, 2- und 4-Allylpyridin, und die Salze davon.

Geeignete Monomere e) sind auch N-Vinylimidazole der allgemeinen Formel (I), worin R¹ bis R³ für Wasserstoff, C₁-C₄-Alkyl oder Phenyl steht

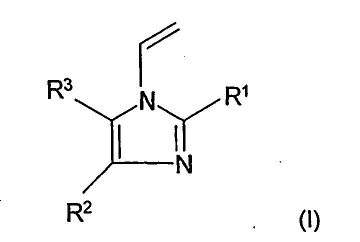

Beispiele für Verbindungen der allgemeinen Formel (I) sind folgender Tabelle 1 zu entnehmen:

**Tabelle 1**

| **R¹** | **R²** | **R³** |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl Ph = Phenyl | | |

Besonders bevorzugt sind die Verbindungen der Komponente e) ausgewählt unter N-(tert.-Butylamino)ethyl(meth)acrylat, N,N-Dimethylaminoethyl(meth)acrylat, N-[3-(dimethylamino)propyl](meth)acrylamid, Vinylimidazol und Mischungen davon.

Die erfindungsgemäßen Copolymere enthalten vorzugsweise bis zu 25 Gew.-%, besonders bevorzugt bis zu 20 Gew.-%, insbesondere bis zu 10 Gew.-%, bezogen auf das Gesamtgewicht der zur Polymerisation eingesetzten Monomere, wenigstens ein Monomer e) einpolymerisiert. Wenn ein Monomer e) eingesetzt wird, dann vorzugsweise in einer Menge von mindestens 1 Gew.-%, besonders bevorzugt mindestens 2 Gew.-%.

### Monomer f)

Die erfindungsgemäßen Copolymere können zusätzlich wenigstens ein von den Komponenten a) bis e) verschiedenes, damit copolymerisierbares Monomer f) einpolymerisiert enthalten.

Vorzugsweise ist die Komponente f) ausgewählt unter von Komponente a) verschiedenen Estern α,β-ethylenisch ungesättigter Mono- und Dicarbonsäuren mit C₁-C₃₀-Alkanolen und C₁-C₃₀-Alkandiolen, Amiden α,β-ethylenisch ungesättigter Mono- und Dicarbonsäuren mit C₂-C₃₀-Aminoalkoholen, die eine primäre oder sekundäre Aminogruppe aufweisen, N-Vinyllactamen, N-Vinylamiden gesättigter Monocarbonsäuren, von Komponente b) verschiedenen primären Amiden α,β-ethylenisch ungesättigter Monocarbonsäuren und deren N-Alkyl- und N,N-Dialkylderivaten, Estern von Vinylalkohol und Allylalkohol mit C₁-C₃₀-Monocarbonsäuren, Vinylethern, Vinylaromaten, Vinylhalogeniden, Vinylidenhalogeniden, C₁-C₈-Monoolefinen, nicht aromatischen Kohlenwasserstoffen mit mindestens zwei konjugierten Doppelbindungen und Mischungen davon.

Als Monomere f) geeignete N-Vinyllactame sind unsubstituierte N-Vinyllactame und N-Vinyllactamderivate, die z. B. einen oder mehrere C₁-C₆-Alkylsubstituenten, wie Methyl, Ethyl, n-Propyl, Isopropyl, n-Butyl, sec.-Butyl, tert.-Butyl etc., aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc. In einer bevorzugten Ausführung enthalten die erfindungsgemäßen Copolymere keine N-Vinyllactame einpolymerisiert.

Als Monomere f) geeignete N-Vinylamidverbindungen sind beispielsweise N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid und N-Vinylbutyramid. In einer bevorzugten Ausführung enthalten die erfindungsgemäßen Copolymere keine N-Vinylamidverbindungen einpolymerisiert.

Geeignete zusätzliche Monomere f) sind weiterhin 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat und 3-Hydroxy-2-ethylhexylmethacrylat.

Geeignete zusätzliche Monomere f) sind weiterhin 2-Hydroxyethylacrylamid, 2-Hydroxyethylmethacrylamid, 2-Hydroxyethylethacrylamid, 2-Hydroxypropylacrylamid, 2-Hydroxypropylmethacrylamid, 3-Hydroxypropylacrylamid, 3-Hydroxypropylmethacrylamid, 3-Hydroxybutylacrylamid, 3-Hydroxybutylmethacrylamid, 4-Hydroxybutylacrylamid, 4-Hydroxybutylmethacrylamid, 6-Hydroxyhexylacrylamid, 6-Hydroxyhexylmethacrylamid, 3-Hydroxy-2-ethylhexylacrylamid und 3-Hydroxy-2-ethylhexylmethacrylamid.

Geeignete Monomere f) sind auch Polyetheracrylate, worunter im Rahmen dieser Erfindung allgemein Ester α,β-ethylenisch ungesättigter Mono- und Dicarbonsäuren mit Polyetherolen verstanden werden. Geeignete Polyetherole sind lineare oder verzweigte, endständige Hydroxylgruppen aufweisende Substanzen, die Etherbindungen enthalten. Im Allgemeinen weisen sie ein Molekulargewicht im Bereich von etwa 150 bis 20000 auf. Geeignete Polyetherole sind Polyalkylenglycole, wie Polyethylenglycole, Polypropylenglycole, Polytetrahydrofurane und Alkylenoxidcopolymere. Geeignete Alkylenoxide zur Herstellung von Alkylenoxidcopolymeren sind z. B. Ethylenoxid, Propylenoxid, Epichlorhydrin, 1,2- und 2,3-Butylenoxid. Die Alkylenoxidcopolymere können die Alkylenoxideinheiten statistisch verteilt oder in Form von Blöcken einpolymerisiert enthalten. Bevorzugt sind Ethylenoxid/Propylenoxid-Copolymere.

Bevorzugt als Komponente f) sind Polyetheracrylate der allgemeinen Formel II 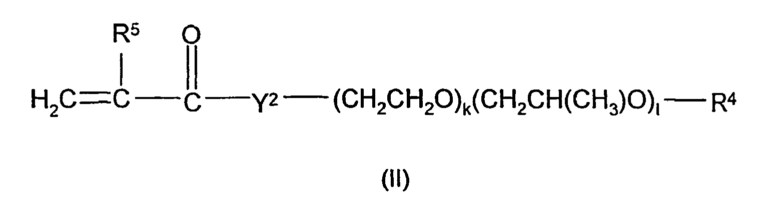 worin
die Reihenfolge der Alkylenoxideinheiten beliebig ist,
- k und l: unabhängig voneinander für eine ganze Zahl von 0 bis 1000 stehen, wobei die Summe aus k und l mindestens 5 beträgt,
- R⁴: für Wasserstoff, C₁-C₃₀-Alkyl oder C₅-C₈-Cycloalkyl steht,
- R⁵: für Wasserstoff oder C₁-C₈-Alkyl steht,
- Y²: für O oder NR⁶ steht, wobei R⁶ für Wasserstoff, C₁-C₃₀-Alkyl oder C₅-C₈-Cycloalkyl steht,

Bevorzugt steht k für eine ganze Zahl von 1 bis 500, insbesondere 3 bis 250. Bevorzugt steht I für eine ganze Zahl von 0 bis 100.

Bevorzugt steht R⁵ für Wasserstoff, Methyl, Ethyl, n-Propyl, Isopropyl, n-Butyl, sek.-Butyl, tert.-Butyl, n-Pentyl oder n-Hexyl, insbesondere für Wasserstoff, Methyl oder Ethyl.

Vorzugsweise steht R⁴ in der Formel II für Wasserstoff, Methyl, Ethyl, n-Propyl, Isopropyl, n-Butyl, sek.-Butyl, n-Pentyl, n-Hexyl, Octyl, 2-Ethylhexyl, Decyl, Lauryl, Palmityl oder Stearyl.

Vorzugsweise steht Y² in der Formel II für O oder NH.

Geeignete Polyetheracrylate f) sind z. B. die Polykondensationsprodukte der zuvor genannten α,β-ethylenisch ungesättigten Mono- und/oder Dicarbonsäuren und deren Säurechloriden, -amiden und Anhydriden mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol R⁴-OH hergestellt werden. Die Alkylenoxide können einzeln, alternierend nacheinander oder als Mischung eingesetzt werden. Die Polyetheracrylate e) können allein oder in Mischungen zur Herstellung der erfindungsgemäß eingesetzten Polymere verwendet werden.

Geeignete zusätzliche Monomere f) sind Methyl(meth)acrylat, Methylethacrylat, Ethyl(meth)acrylat, 2-Pentyl(meth)acrylat, 3-Pentyl(meth)acrylat, Isopentylacrylat, Neopentylacrylat, Ethylethacrylat, Isopropyl(meth)acrylat, n-Butyl(meth)acrylat, sek.-Butyl(meth)acrylat, Isobutyl(meth)acrylat, tert.-Butylethacrylat, n-Octyl(meth)acrylat, 1,1,3,3-Tetramethylbutyl(meth)acrylat, Ethylhexyl(meth)acrylat, n-Nonyl(meth)acrylat, n-Decyl(meth)acrylat, n-Undecyl(meth)acrylat, Tridecyl(meth)acrylat, Myristyl(meth)acrylat, Pentadecyl(meth)acrylat, Palmityl(meth)acrylat, Heptadecyl(meth)acrylat, Nonadecyl(meth)acrylat, Arrachinyl(meth)acrylat, Behenyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Linolyl(meth)acrylat, Linolenyl(meth)acrylat, Stearyl(meth)acrylat, Lauryl(meth)acrylat und Mischungen davon. Bevorzugte Monomere e) sind die Ester α,β-ethylenisch ungesättigter Mono- und Dicarbonsäuren mit C₁-C₄-Alkanolen.

Geeignete zusätzliche Monomere f) sind weiterhin N-(n-Butyl)methacrylamid, N-(sek.-Butyl)methacrylamid, N-(tert.-Butyl)methacrylamid, N-(n-Pentyl)(meth)acrylamid, N-(n-Hexyl)(meth)acrylamid, N-(n-Heptyl)(meth)acrylamid, N-(n-Octyl)(meth)acrylamid, N-(tert.-Octyl)(meth)acrylamid N-(1,1,3,3-Tetramethylbutyl)(meth)acrylamid, N-Ethylhexyl(meth)acrylamid, N-(n-Nonyl)(meth)acrylamid, N-(n-Decyl)(meth)acrylamid, N-(n-Undecyl)(meth)acrylamid, N-Tridecyl(meth)acrylamid, N-Myristyl(meth)acrylamid, N-Pentadecyl(meth)acrylamid, N-Palmityl(meth)acrylamid, N-Heptadecyl(meth)acrylamid, N-Nonadecyl(meth)acrylamid, N-Arrachinyl(meth)acrylamid, N-Behenyl(meth)acrylamid, N-Lignocerenyl(meth)acrylamid, N-Cerotinyl(meth)acrylamid, N-Melissinyl(meth)acrylamid, N-Palmitoleinyl(meth)acrylamid, N-Oleyl(meth)acrylamid, N-Linolyl(meth)acrylamid, N-Linolenyl(meth)acrylamid, N-Stearyl(meth)acrylamid, N-Lauryl(meth)acrylamid.

Geeignete zusätzliche Monomere f) sind weiterhin Vinylacetat, Vinylpropionat, Vinylbutyrat und Mischungen davon.

Geeignete zusätzliche Monomere f) sind weiterhin Ethylen, Propylen, Isobutylen, Butadien, Styrol, α-Methylstyrol, Acrylnitril, Methacrylnitril, Vinylchlorid, Vinylidenchlorid, Vinylfluorid, Vinylidenfluorid und Mischungen davon.

Die zuvor genannten zusätzlichen Monomere f) können einzeln oder in Form von beliebigen Mischungen eingesetzt werden.

Bevorzugt enthalten die erfindungsgemäßen Copolymere wenigstens eine Verbindung f) einpolymerisiert, die ausgewählt ist unter C₁-C₃-Alkylmethacrylaten, Hydroxy-C₁-C₃-alkylmethacrylaten und Mischungen davon. Besonders bevorzugt sind Ethylmethacrylat, Hydroxyethylmethacrylat und Mischungen davon. Insbesondere wird Ethylmethacrylat eingesetzt. Die erfindungsgemäßen Copolymere enthalten diese Monomere vorzugsweise in einer Menge von höchstens 50 Gew.-%, besonders bevorzugt höchstens 45 Gew.-%, bezogen auf das Gesamtgewicht aus Verbindungen der Komponente a) und diesen Verbindungen f), einpolymerisiert.

Die erfindungsgemäßen Copolymere enthalten vorzugsweise bis zu 25 Gew.-%, besonders bevorzugt bis zu 20 Gew.-%, insbesondere bis zu 15 Gew.-%, bezogen auf das Gesamtgewicht der zur Polymerisation eingesetzten Monomere, wenigstens eines Monomers f) einpolymerisiert. Wenn ein Monomer f) eingesetzt wird, dann vorzugsweise in einer Menge von mindestens 0,1 Gew.%, besonders bevorzugt mindestens 1 Gew.-% und insbesondere wenigstens 5 Gew.-%.

### Vernetzer g)

Die erfindungsgemäßen Copolymere können gewünschtenfalls wenigstens einen Vernetzer, d. h. eine Verbindung mit zwei oder mehr als zwei ethylenisch ungesättigten, nichtkonjugierten Doppelbindungen einpolymerisiert enthalten.

Vorzugsweise werden Vernetzer in einer Menge von 0,01 bis 3 Gew.-%, besonders bevorzugt 0,1 bis 2 Gew.%, bezogen auf das Gesamtgewicht der zur Polymerisation eingesetzten Monomere, verwendet.

Geeignete Vernetzer g) sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei ethylenisch ungesättigte Gruppen.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer g) sind die Vinylester oder die Ester einwertiger, ungesättigter Alkohole mit ethylenisch ungesättigten C₃-C₆-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellithsäure, Phthalsäure, Terephthalsäure, Zitronensäure oder Bernsteinsäure.

Weitere geeignete Vernetzer g) sind Ester ungesättigter Carbonsäuren mit den oben beschriebenen mehrwertigen Alkoholen, beispielsweise der Ölsäure, Crotonsäure, Zimtsäure oder 10-Undecensäure.

Weitere geeignete Vernetzer g) sind Urethandiacrylate und Urethanpolyacrylate, wie sie z. B. unter der Bezeichnung Laromer® kommerziell erhältlich sind.

Geeignet als Vernetzer g) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z. B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Vernetzer g) sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren, wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z. B. Triallylmethylammoniumchlorid oder -methylsulfat, als Vernetzer g) geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z. B. N,N'-Divinylethylenharnstoff oder N,N'-Divinylpropylenharnstoff.

Weitere geeignete Vernetzer g) sind Divinyldioxan, Tetraallylsilan oder Tetravinylsilan.

Selbstverständlich können auch Mischungen der vorgenannten Verbindungen g) eingesetzt werden. Vorzugsweise werden wasserlösliche Vernetzer g) eingesetzt.

Besonders bevorzugt eingesetzte Vernetzer g) sind beispielsweise Methylenbisacrylamid, Triallylamin und Triallylalkylammoniumsalze, Divinylimidazol, Pentaerythrittriallylether, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer g) sind Pentaerythrittriallylether, Methylenbisacrylamid, N,N'-Divinylethylenharnstoff, Triallylamin und Triallylmonoalkylammoniumsalze und Acrylsäureester von Glykol, Butandiol, Trimethylolpropan oder Glycerin oder Acrylsäureester von mit Ethylenoxid und/oder Epichlorhydrin umgesetztem Glykol, Butandiol, Trimethylolpropan oder Glycerin.

Bevorzugt sind Copolymere, die
- 30 bis 90 Gew.-%, besonders bevorzugt 40 bis 85 Gew.-%, wenigstens einer Verbindung a),
- 3 bis 50 Gew.-%, besonders bevorzugt 5 bis 40 Gew.-%, insbesondere 10 bis 35 Gew.-%, wenigstens einer Verbindung b),
- 5 bis 40 Gew.-%, besonders bevorzugt 7 bis 35 Gew.-%, insbesondere 10 bis 30 Gew.-%, Acrylsäure c),
- 0 bis 25 Gew.-%, besonders bevorzugt 1 bis 20 Gew.-%, insbesondere 5 bis 15 Gew.-%, Methacrylsäure d),
- 0 bis 25 Gew.-%, besonders bevorzugt 1 bis 20 Gew.-%, insbesondere 2 bis 10 Gew.-%, wenigstens einer Verbindung e);
- 0 bis 25 Gew.-%, besonders bevorzugt 0,1 bis 20 Gew.-%, insbesondere 5 bis 15 Gew.-%, wenigstens einer Verbindung f),
- 0 bis 5 Gew.-%, besonders bevorzugt 0,01 bis 3 Gew.-%, insbesondere 0,1 bis 2 Gew.-%, wenigstens eines Vernetzers g),
einpolymerisiert enthalten.

Eine bevorzugte Ausführung sind Copolymere, die aus Wiederholungseinheiten von
- tert.-Butylacrylat,
- Methacrylsäureamid und/oder N-(tert.-Butyl)acrylamid, und
- Acrylsäure
bestehen.

Eine weitere bevorzugte Ausführung sind Copolymere, die aus Wiederholungseinheiten von
- tert.-Butylacrylat,
- Methacrylsäureamid und/oder N-(tert.-Butyl)acrylamid,
- Acrylsäure und
- Methacrylsäure
   bestehen.

Eine weitere bevorzugte Ausführung sind Copolymere, die aus Wiederholungseinheiten von
- tert.-Butylacrylat,
- N-Acryloylmorpholin oder N,N-Dimethylacrylamid und
- Acrylsäure
   bestehen.

Eine weitere bevorzugte Ausführung sind Copolymere, die aus Wiederholungseinheiten von
- tert.-Butylacrylat,
- Methacrylsäureamid und/oder N-(tert.-Butyl)acrylamid und/oder N-Acryloylmorpholin und/oder N,N-Dimethylacrylamid und
- Acrylsäure
bestehen.

Eine weitere bevorzugte Ausführung sind Copolymere, die aus Wiederholungseinheiten von
- tert.-Butylacrylat,
- Methacrylsäureamid und/oder N-(tert.-Butyl)acrylamid und/oder N-Acryloylmorpholin und/oder N,N-Dimethylacrylamid,
- Acrylsäure und
- Methacrylsäure
bestehen.

Eine weitere bevorzugte Ausführung sind Copolymere, die aus Wiederholungseinheiten von
- tert.-Butylacrylat,
- Methacrylsäureamid und/oder N-(tert.-Butyl)acrylamid,
- N-Vinylpyrrolidon und
- Acrylsäure
   bestehen.

Eine weitere bevorzugte Ausführung sind Copolymere, die aus Wiederholungseinheiten von
- tert.-Butylacrylat,
- Methacrylsäureamid und/oder N-(tert.-Butyl)acrylamid,
- N-Vinylpyrrolidon,
- Acrylsäure und
- Methacrylsäure
bestehen.

Eine weitere bevorzugte Ausführung sind Copolymere, die aus Wiederholungseinheiten von
- tert.-Butylacrylat,
- tert.-Butylmethacrylat,
- Methacrylsäureamid und/oder N-(tert.-Butyl)acrylamid, und
- Acrylsäure
bestehen.

Eine weitere bevorzugte Ausführung sind Copolymere, die aus Wiederholungseinheiten von
- tert.-Butylacrylat,
- tert.-Butylmethacrylat,
- Methacrylsäureamid und/oder N-(tert.-Butyl)acrylamid,
- Acrylsäure und
- Methacrylsäure
bestehen.

Eine weitere bevorzugte Ausführung sind Copolymere, die aus Wiederholungseinheiten von
- tert.-Butylacrylat und/oder tert.-Butylmethacrylat,
- Methacrylsäureamid und/oder N-(tert.-Butyl)acrylamid,
- Acrylsäure und
- N-(tert.-Butyl)aminoethyl(meth)acrylat oder N-[3-(dimethylamino)propyl]methacrylamid oder N-Vinylimidazol
bestehen.

Eine weitere bevorzugte Ausführung sind Copolymere, die aus Wiederholungseinheiten von
- tert.-Butylacrylat und/oder tert.-Butylmethacrylat,
- Methacrylsäureamid und/oder N-(tert.-Butyl)acrylamid,
- Acrylsäure,
- Methacrylsäure und
- N-(tert.-Butyl)aminoethyl(meth)acrylat oder N-[3-(dimethylamino)propyl]methacrylamid oder N-Vinylimidazol
bestehen.

Bevorzugt sind weiterhin Copolymere, die
a) tert.-Butylacrylat und/oder tert.-Butylmethacrylat,
b) Methacrylsäureamid und/oder N-(tert.-Butyl)acrylamid,
c) Acrylsäure und
f) wenigstens eine Verbindung, die ausgewählt ist unter C₁-C₃-Alkylmethacrylaten, Hydroxy-C₁-C₃-alkylmethacrylaten und Mischungen davon,
einpolymerisiert enthalten, mit der Maßgabe, dass der Gewichtsmengenanteil der Komponente a) gleich ist wie oder größer ist als der Gewichtsmengenanteil der Komponente f).

Eine bevorzugte Ausführung sind Copolymere, die
a) tert.-Butylacrylat,
b) Methacrylsäureamid und/oder N-(tert.-Butyl)acrylamid,
c) Acrylsäure,
d) Methacrylsäure,
f) Ethylmethacrylat und/oder Hydroxyethylmethacrylat
einpolymerisiert enthalten, mit der Maßgabe, dass der Gewichtsmengenanteil der Komponente a) gleich ist wie oder größer ist als der Gewichtsmengenanteil der Komponente f).

Eine besonders bevorzugte Ausführung sind Copolymere, die aus Wiederholungseinheiten von
- tert.-Butylacrylat,
- Methacrylsäureamid und/oder N-(tert.-Butyl)acrylamid,
- Acrylsäure und
- Ethylmethacrylat
   bestehen, mit der Maßgabe, dass der Gewichtsmengenanteil an tert.-Butylacrylat gleich ist wie oder größer ist als der Gewichtsmengenanteil an Ethylmethacrylat.

Eine weitere besonders bevorzugte Ausführung sind Copolymere, die aus Wiederholungseinheiten von
- tert.-Butylacrylat,
- Methacrylsäureamid und/oder N-(tert.-Butyl)acrylamid,
- Acrylsäure
- Methacrylsäure und
- Ethylmethacrylat
bestehen, mit der Maßgabe, dass der Gewichtsmengenanteil an tert.-Butylacrylat gleich ist wie oder größer ist als der Gewichtsmengenanteil an Ethylmethacrylat.

Bevorzugt sind Copolymere, die, jeweils bezogen auf das Gesamtgewicht der zur Polymerisation eingesetzten Monomere,
- 40 bis 80 Gew.-%, bevorzugt 45 bis 75 Gew.-%, tert.-Butylacrylat und Ethyl-methacrylat, mit der Maßgabe, dass der Gewichtsanteil an tert.-Butylmethacrylat gleich ist wie oder größer ist als der Gewichtsmengenanteil von Ethylmethacrylat,
- 5 bis 30 Gew.-%, bevorzugt 7 bis 25 Gew.-%, Methacrylsäureamid und/oder N-(tert.-Butyl)acrylamid,
- 5 bis 30 Gew.-%, bevorzugt 5 bis 15 Gew.-%, Acrylsäure und
- 0 bis 25 Gew.-%, bevorzugt 5 bis 20 Gew.-%, Methacrylsäure
   einpolymerisiert enthalten.

Die Herstellung der erfindungsgemäßen Copolymere kann z. B. durch Lösungs-, Fällungs-, Suspensions- oder Emulsionspolymerisation erfolgen. Derartige Verfahren sind dem Fachmann prinzipiell bekannt. Bevorzugt ist die Herstellung durch Lösungspolymerisation.

Bevorzugte Lösemittel zur Polymerisation sind wässrige Lösungsmittel, wie Wasser und Gemische aus Wasser mit wassermischbaren Lösungsmitteln, beispielsweise Alkoholen, wie Methanol, Ethanol, n-Propanol, Isopropanol, n-Butanol, sek.-Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Polyethylenglykolen mit zahlenmittleren Molekulargewichten bis etwa 3000, Glycerin und Dioxan. Besonders bevorzugt ist die Polymerisation in Wasser oder einem Wasser/Alkohol-Gemisch, beispielsweise in einem Wasser/Ethanol-Gemisch.

Die Polymerisation kann prinzipiell bei dem durch die eingesetzten Monomere resultierenden pH-Wert erfolgen. Wird zur Polymerisation wenigstens ein N-Vinyllactam eingesetzt (= Komponente f)), so wird der pH-Wert des Polymerisationsmediums vorzugsweise auf einen Wert von 5 bis 8, bevorzugt 6 bis 7, eingestellt. Es ist vorteilhaft, den pH-Wert während der Polymerisation dann in diesem Bereich zu halten. Zur Einstellung des pH-Werts vor, während oder nach der Polymerisation eignen sich prinzipiell alle anorganischen oder organischen Basen (und gegebenenfalls Säuren), insbesondere solche, die außer einer etwaigen Salzbildung keine Reaktion mit den Monomeren eingehen. Geeignete Basen sind z. B. Alkali- und Erdalkalihydroxide, Ammoniak sowie primäre, sekundäre und tertiäre Amine, wie Triethylamin, sowie Aminoalkohole, wie Triethanolamin, Methyldiethanolamin, Dimethylethanolamin oder 2-Amino-2-methylpropanol. Bevorzugt wird zur Einstellung des pH-Werts wenigstens ein tertiären Amin, das insbesondere ausgewählt ist unter N,N-Dimethylethanolamin, N-Methyldiethanolamin, Triethanolamin und Mischungen davon, eingesetzt. Wird zur Polymerisation wenigstens ein N-Vinyllactam eingesetzt (= Komponente f)), so wird der pH-Wert des Polymerisationsmediums vorzugsweise mit N,N-Dimethylethanolamin eingestellt.

Die Polymerisationstemperaturen liegen vorzugsweise in einem Bereich von etwa 30 bis 120 °C, besonders bevorzugt 40 bis 100 °C. Die Polymerisation erfolgt üblicherweise unter atmosphärischem Druck, sie kann jedoch auch unter vermindertem oder erhöhtem Druck ablaufen. Ein geeigneter Druckbereich liegt zwischen 1 und 5 bar.

Zur Copolymerisation können die Monomeren mit Hilfe von Radikale bildenden Initiatoren polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid,
Di-tert.-butylperoxid, tert.-Butylperbenzoat, tert.-Butylperpivalat,
tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat,
Di-tert.-Amylperoxid, tert.-Butylhydroperoxid, Azobisisobutyronitril,
2,2'-Azobis(2-amidinopropan)hydrochloride (V50 von Wako Pure Chemicals Industries, Ltd.), oder 2,2'-Azobis(2-methyl-butyronitril). Geeignet sind auch Initiatormischungen oder Redox-Initiator-Systeme, wie z. B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat, H₂O₂/Cu¹.

Zur Einstellung des Molekulargewichts kann die Polymerisation in Gegenwart wenigstens eines Reglers erfolgen. Als Regler können die üblichen, dem Fachmann bekannten Verbindungen, wie z. B. Schwefelverbindungen, z. B. Mercaptoethanol, 2-Ethylhexylthioglycolat, Thioglycolsäure oder Dodecylmercaptan sowie Tribromchlormethan oder andere Verbindungen, die regelnd auf das Molekulargewicht der erhaltenen Polymerisate wirken, eingesetzt werden. Ein bevorzugter Regler ist Cystein.

Zur Erzielung möglichst reiner Polymere mit geringem Restmonomergehalt kann sich an die Polymerisation (Hauptpolymerisation) ein Nachpolymerisationsschritt anschließen. Die Nachpolymerisation kann in Gegenwart desselben oder eines anderen Initiatorsystems wie die Hauptpolymerisation erfolgen. Vorzugsweise erfolgt die Nachpolymerisation mindestens bei der gleichen, vorzugsweise bei einer höheren Temperatur als die Hauptpolymerisation. Gewünschtenfalls kann der Reaktionsansatz im Anschluss an die Polymerisation oder zwischen dem ersten und dem zweiten Polymerisationsschritt einem Strippen mit Wasserdampf oder einer Wasserdampf-Destillation unterzogen werden.

Wird bei der Herstellung der Polymere ein organisches Lösungsmittel eingesetzt, so kann dieses durch übliche, dem Fachmann bekannte Verfahren, z. B. durch Destillation bei vermindertem Druck, entfernt werden.

Die erhaltenen flüssigen Polymerzusammensetzungen können durch verschiedene Trocknungsverfahren, wie z. B. Sprühtrocknung, Fluidized Spray Drying, Walzentrocknung oder Gefriertrocknung in Pulverform überführt werden. Bevorzugt wird die Sprühtrocknung eingesetzt. Die so erhaltenen Polymer-Trockenpulver lassen sich vorteilhafterweise durch Lösen bzw. Redispergieren in Wasser erneut in eine wässrige Lösung bzw. Dispersion überführen. Pulverförmige Copolymere haben den Vorteil einer besseren Lagerfähigkeit, einer einfacheren Transportmöglichkeit und zeigen in der Regel eine geringere Neigung für Keimbefall. Dazu zählen ganz allgemein anionische, kationische, amphotere und neutrale Polymere.

Die zuvor beschriebenen Copolymere eignen sich hervorragend zur Herstellung kosmetischer und pharmazeutischer Mittel. Sie dienen dabei z. B. als polymere Filmbildner in Zubereitungen für die Körperpflege, was die Anwendung in kosmetischen Zubereitungen für keratinöse Oberflächen wie Haut, Haar, Nägel sowie auch Mundpflegepräparate beinhaltet. Sie sind universell in den verschiedensten kosmetischen Zubereitungen einsetzbar und einformulierbar und mit den üblichen Komponenten verträglich. Insbesondere eignen sich die erfindungsgemäßen Copolymere zur Herstellung haarkosmetischer Mittel. Gegenüber üblichen, aus dem Stand der Technik bekannten Polymeren eignen sie sich vorteilhaft zur Erzeugung elastischer Frisuren bei gleichzeitig starker Festigung (auch bei hoher Luftfeuchtigkeit). Die erfindungsgemäßen Copolymere zeichnen sich zudem durch eine gute Treibgasverträglichkeit, eine gute Löslichkeit in Wasser oder wässrig/alkoholischen Lösungsmittelgemischen, die Eignung für einen Einsatz in Low-VOC-Formulierungen und eine gute Auswaschbarkeit aus. Zudem haben sie in der Regel auch gute konditionierende Eigenschaften, d. h. sie verbessern damit behandeltes Haar in seinen sensorisch erfassbaren Eigenschaften wie Griff, Volumen, Handhabbarkeit usw. Haarsprayformulierungen auf Basis der erfindungsgemäßen Copolymere zeichnen sich durch gute rheologische Eigenschaften und eine gute Sprühbarkeit aus.

### Kosmetisch akzeptabler Träger B)

Die erfindungsgemäßen Mittel weisen einen kosmetisch oder pharmazeutisch akzeptablen Träger B) auf, der ausgewählt ist unter
i) Wasser,
ii) wassermischbaren organischen Lösungsmitteln, vorzugsweise C₂-C₄-Alkanolen, insbesondere Ethanol,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen,
und Mischungen davon.

Die erfindungsgemäßen Mittel weisen z. B. eine Öl- bzw. Fettkomponente B) auf, die ausgewählt ist unter: Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestern; Vaselin; Estern, bevorzugt Estern von Fettsäuren, wie z. B. die Ester von C₁-C₂₄-Monoalkoholen mit C₁-C₂₂-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexancosanylstearat, Octacosanylstearat, Triacontanylstearat, Dotriacontanylstearat, Tetratriacontanylstearat; Salicylaten, wie C₁-C₁₀-Salicylaten, z. B. Octylsalicylat; Benzoatestern, wie C₁₀-C₁₅-Alkylbenzoaten, Benzylbenzoat; anderen kosmetischen Estern, wie Fettsäuretriglyceriden, Propylenglykolmonolaurat, Polyethylenglykolmonolaurat, C₁₀-C₁₅-Alkyllactaten, etc. und Mischungen davon.

Geeignete Siliconöle B) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten B) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete kosmetisch und pharmazeutisch verträgliche Öl- bzw. Fettkomponenten B) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Geeignete hydrophile Träger B) sind ausgewählt unter Wasser, 1-, 2- oder mehrwertigen Alkoholen mit vorzugsweise 1 bis 8 Kohlenstoffatomen, wie Ethanol, n-Propanol, iso-Propanol, Propylenglykol, Glycerin, Sorbit, etc.

Bei den erfindungsgemäßen kosmetischen Mitteln kann es sich um hautkosmetische, haarkosmetische, dermatologische, hygienische oder pharmazeutische Mittel handeln. Aufgrund ihrer filmbildenden Eigenschaften eignen sich die zuvor beschriebenen Copolymere und Polyelektrolytkomplexe insbesondere als Zusatzstoffe für Haar- und Hautkosmetika.

Vorzugsweise liegen die erfindungsgemäßen Mittel in Form eines Sprays, Gels, Schaums, einer Salbe, Creme, Emulsion, Suspension, Lotion, Milch oder Paste vor. Gewünschtenfalls können auch Liposomen oder Mikrosphären eingesetzt werden.

Die erfindungsgemäßen kosmetisch oder pharmazeutisch aktiven Mittel können zusätzlich kosmetisch und/oder dermatologisch aktive Wirkstoffe sowie Hilfsstoffe enthalten.

Vorzugsweise enthalten die erfindungsgemäßen kosmetischen Mittel wenigstens ein wie vorstehend definiertes Copolymer (= Komponente A), wenigstens einen wie vorstehend definierten Träger B) und wenigstens einen davon verschiedenen Bestandteil, der ausgewählt ist unter kosmetisch aktiven Wirkstoffen, Emulgatoren, Tensiden, Konservierungsmitteln, Parfümölen, Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmitteln, Farbstoffen, Pigmenten, Konsistenzgebern, Feuchthaltemitteln, Rückfettern, Collagen, Eiweisshydrolysaten, Lipiden, Antioxidantien, Entschäumern, Antistatika, Emollienzien und Weichmachern.

Übliche Verdickungsmittel in derartigen Formulierungen sind vernetzte Polyacrylsäuren und deren Derivate, Polysaccharide und deren Derivate, wie Xanthan-gum, Agar-Agar, Alginate oder Tylosen, Cellulosederivate, z. B. Carboxymethylcellulose oder Hydroxycarboxymethylcellulose, Fettalkohole, Monoglyceride und Fettsäuren, Polyvinylalkohol und Polyvinylpyrrolidon. Bevorzugt werden nichtionische Verdicker eingesetzt.

Geeignete kosmetisch und/oder dermatologisch aktive Wirkstoffe sind z. B. färbende Wirkstoffe, Haut- und Haarpigmentierungsmittel, Tönungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z. B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-B-und/oder UV-A-Bereich absorbieren. Geeignete UV-Filter sind z. B. 2,4,6-Triaryl-1,3,5-triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl und Mischungen davon. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc. Geeignete hyperemisierend wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Die erfindungsgemäßen kosmetischen Mittel können als kosmetischen und/oder pharmazeutischen Wirkstoff (wie auch gegebenenfalls als Hilfsstoff) wenigstens ein kosmetisch oder pharmazeutisch akzeptables Polymer enthalten.

Als zusätzliche Polymere bevorzugte anionische Polymere sind beispielsweise Homo- und Copolymerisate von Acrylsäure und Methacrylsäure und deren Salze. Dazu zählen auch vernetzte Polymere der Acrylsäure, wie sie unter dem INCI-Namen Carbomer erhältlich sind. Derartige vernetzte Homopolymere der Acrylsäure sind beispielsweise kommerziell unter dem Namen Carbopol® von der Firma BF GOODRICH erhältlich. Bevorzugt sind auch hydrophob modifizierte vernetzte Polyacrylat Polymere, wie Carbopol® Ultrez 21 von der Firma Noveon.

Weitere Beispiele für geeignete zusätzliche anionische Polymere sind Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Besonders geeignete Polymere sind Copolymere aus (Meth)acrylsäure und Polyetheracrylaten, wobei die Polyetherkette mit einem C₈-C₃₀-Alkylrest terminiert ist. Dazu zählen z. B. Acrylat/Beheneth-25-methacrylat-Copolymere, die unter der Bezeichnung Aculyn® von der Firma Rohm und Haas erhältlich sind. Besonders geeignete Polymere sind weiterhin Copolymere aus t-Butylacrylat, Ethylacrylat, Methacrylsäure (z. B. Luvimer® 100P), Copolymere aus Ethylacrylat und Methacrylsäure (z. B. Luviumer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vinylester (z. B. Luviset® Marken), Maleinsäureanhydridcopolymere, gegebenenfalls mit Alkohol umgesetzt, anionische Polysiloxane, z. B. carboxyfunktionelle, t-Butylacrylat, Methacrylsäure (z. B. Luviskol® VBM), Copolymere von Acrylsäure und Methacrylsäure mit hydrophoben Monomeren, wie z. B. C₄-C₃₀-Alkylester der Meth(acrylsäure), C₄-C₃₀-Alkylvinylester, C₄-C₃₀-Alkylvinylether und Hyaluronsäure. Beispiele für anionische Polymere sind weiterhin Vinylacetat/Crotonsäure-Copolymere, wie sie beispielsweise unter den Bezeichnungen Resyn® (National Starch) und Gafset® (GAF) im Handel sind und Vinylpyrrolidon/Vinylacrylat-Copolymere, erhältlich beispielsweise unter dem Warenzeichen Luviflex® (BASF). Weitere geeignete Polymere sind das unter der Bezeichnung Luviflex® VBM-35 (BASF) erhältliche Vinylpyrrolidon/Acrylat-Terpolymer und Natriumsulfonat-haltige Polyamide oder Natriumsulfonat-haltige Polyester.

Weiterhin umfasst die Gruppe der geeigneten anionischen Polymere beispielhaft Balance® CR (National Starch; Acrylate Copolymer), Balance® 0/55 (National Starch; Acrylate Copolymer), Balance® 47 (National Starch; Octylacrylamid/Acrylate/Butylaminoethylmethacrylate-Copolymer), Aquaflex® FX 64 (ISP; Isobutylen/Ethylmaleimid/Hydroxyethylmaleimid-Copolymer), Aquaflex® SF-40 (ISP / National Starch; VP/Vinyl Caprolactam/DMAPA Acrylate Copolymer), Allianz® LT-1 20 (ISP; Rohm & Haas; Acrylat/C1-2 Succinat/Hydroxyacrylat Copolymer), Aquarez® HS (Eastman; Polyester-1), Diaformer® Z-400 (Clariant; Methacryloylethylbetain/Methacrylat-Copolymer), Diaformer® Z-711 (Clariant; Methacryloylethyl N-oxid/Methacrylat-Copolymer), Diaformer® Z-712 (Clariant; Methacryloylethyl N-oxide/Methacrylat-Copolymer), Omnirez® 2000 (ISP; Monoethylester von Poly(Methylvinylether/Maleinsäure in Ethanol), Amphomer® HC (National Starch; Acrylat/ Octylacrylamid-Copolymer), Amphomer® 28-4910 (National Starch; Octylacrylamid/Acrylat/Butylaminoethylmethacrylat-Copolymer), Advantage® HC 37 (ISP; Terpolymer aus Vinylcaprolactam/Vinylpyrrolidon/Dimethylaminoethylmethacrylat), Advantage® LC55 und LC80 oder LC A und LC E, Advantage® Plus (ISP; VA/Butyl Maleatellsobornyl Acrylate Copolymer), Aculyne® 258 (Rohm & Haas; Acrylat/ Hydroxyesteracrylat-Copolymer), Luviset® P.U.R. (BASF, Polyurethane-1), Luviflex® Silk (BASF), Eastman® AQ 48 (Eastman), Styleze® CC-10 (ISP; VP/DMAPA Acrylates Copolymer), Styleze® 2000 (ISP; VP/Acrylates/Lauryl Methacrylate Copolymer), DynamX (National Starch; Polyurethane-14 AMP-Acrylates Copolymer), Resyn XP (National Starch; Acrylates/Octylacrylamide Copolymer), Fixomer A-30 (Ondeo Nalco; Polymethacrylsäure (und) Acrylamidomethylpropansulfonsäure), Fixate G-100 (Noveon; AMP-Acrylates/Allyl Methacrylate Copolymer).

Geeignete zusätzliche Polymere sind auch die in der US 3,405,084 beschriebenen Terpolymere aus Vinylpyrrolidon, C₁-C₁₀-Alkyl- Cycloalkyl- und Aryl(meth)acrylaten und Acrylsäure. Geeignete zusätzliche Polymere sind weiterhin die in der EP-A-0 257 444 und EP-A-0 480 280 beschriebenen Terpolymere aus Vinylpyrrolidon, tert.-Butyl(meth)acrylat und (Meth)acrylsäure. Geeignete zusätzliche Polymere sind weiterhin die in der DE-A-42 23 066 beschriebenen Copolymere, die wenigstens einen (Meth)acrylsäureester, (Meth)acrylsäure sowie N-Vinylpyrrolidon und/oder N-Vinylcaprolactam einpolymerisiert enthalten. Auf die Offenbarung dieser Dokumente wird hier Bezug genommen.

Geeignete Carbonsäuregruppen-haltige Polymere sind weiterhin Carbonsäuregruppen-haltige Polyurethane.

Die EP-A-636361 offenbart geeignete Blockcopolymere mit Polysiloxanblöcken und Polyurethan-/Polyharnstoffblöcken, die Carbonsäure- und/oder Sulfonsäuregruppen aufweisen. Geeignete siliconhaltige Polyurethane sind auch in der WO 97/25021 und der EP-A-751 162 beschrieben. Geeignete Polyurethane sind auch in der DE-A-42 25 045 beschrieben, worauf hier in vollem Umfang Bezug genommen wird. Diese Polyurethane sind prinzipiell aufgebaut aus
i) mindestens einer Verbindung, die zwei oder mehrere aktive Wasserstoffatome pro Molekül enthält,
ii) mindestens einem Carbonsäuregruppen enthaltenden Diol oder einem Salz davon und
iii) mindestens einem Polyisocyanat.

Bei der Komponente i) handelt es sich z. B. um Diole, Diamine, Aminoalkohole, und Mischungen davon. Das Molekulargewicht dieser Verbindungen liegt vorzugsweise in einem Bereich von etwa 56 bis 280. Gewünschtenfalls können bis zu 3 Mol-% der genannten Verbindungen durch Triole oder Triamine ersetzt sein.

Brauchbare Diole i) sind z. B. Ethylenglykol, Propylenglykol, Butylenglykol, Neopentylglykol, Cyclohexandimethylol, Di-, Tri-, Tetra-, Penta- oder Hexaethylenglykol und Mischungen davon. Bevorzugt werden Neopentylglykol und/oder Cyclohexandimethylol eingesetzt. Geeignete Aminoalkohole i) sind z. B. 2-Aminoethanol, 2-(N-Methylamino)ethanol, 3-Aminopropanol, 4-Aminobutanol, 1-Ethylaminobutan-2-ol, 2-Amino-2-methyl-1-propanol, 4-Methyl-4-aminopentan-2-ol etc. Geeignete Diamine i) sind z. B. Ethylendiamin, Propylendiamin, 1,4-Diaminobutan, 1,5-Diaminopentan und 1,6-Diaminohexan sowie α,ω-Diaminopolyether, die durch Aminierung von Polyalkylenoxiden mit Ammoniak herstellbar sind.

Bei der Komponente i) kann es sich auch um ein Polymerisat mit einem zahlenmittleren Molekulargewicht im Bereich von etwa 300 bis 5000, bevorzugt etwa 400 bis 4000, insbesondere 500 bis 3000. Brauchbare Polymerisate i) sind z. B. Polyesterdiole, Polyetherole und Mischungen davon. Polyetherole sind vorzugsweise Polyalkylenglykole, z. B. Polyethylenglykole, Polypropylenglykole, Polytetrahydrofurane etc., Blockcopolymerisate aus Ethylenoxid und Propylenoxid oder Blockcopolymerisate aus Ethylenoxid, Propylenoxid und Butylenoxid, die die Alkylenoxideinheiten statistisch verteilt oder in Form von Blöcken einpolymerisiert enthalten. Geeignete Polytetrahydrofurane i) können durch kationische Polymerisation von Tetrahydrofuran in Gegenwart von sauren Katalysatoren, wie z. B. Schwefelsäure oder Fluoroschwefelsäure, hergestellt werden. Derartige Herstellungsverfahren sind dem Fachmann bekannt. Brauchbare Polyesterdiole i) weisen vorzugsweise ein zahlenmittleres Molekulargewicht im Bereich von etwa 400 bis 5000, bevorzugt 500 bis 3000, insbesondere 600 bis 2000, auf. Als Polyesterdiole i) kommen alle diejenigen in Betracht, die üblicherweise zur Herstellung von Polyurethanen eingesetzt werden, insbesondere solche auf Basis aromatischer Dicarbonsäuren, wie Terephthalsäure, Isophthalsäure, Phthalsäure, Na- oder K-Sulfoisophthalsäure etc., aliphatischer Dicarbonsäuren, wie Adipinsäure oder Bernsteinsäure etc., und cycloaliphatischer Dicarbonsäuren, wie 1,2-, 1,3- oder 1,4-Cyclohexandicarbonsäure. Als Diole kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan, etc.

Geeignete Verbindungen ii), die zwei aktive Wasserstoffatome und mindestens eine Carbonsäuregruppe pro Molekül aufweisen, sind z. B. Dimethylolpropansäure und Mischungen, die Dimethylolpropansäure enthalten.

Bei der Komponente iii) handelt es sich um übliche aliphatische, cycloaliphatische und/oder aromatische Polyisocyanate, wie Tetramethylendiisocyanat, Hexamethylendiisocyanat, Methylendiphenyldiisocyanat, 2,4- und 2,6-Toluylendiisocyanat und deren Isomerengemische, o- und m-Xylylendiisocyanat, 1,5-Naphthylendiisocyanat, 1,4-Cyclohexylendiisocyanat, Dicyclohexylmethandiisocyanat und Mischungen davon, insbesondere Isophorondiisocyanat und/oder Dicyclohexylmethandiisocyanat. Gewünschtenfalls können bis zu 3 Mol-% der genannten Verbindungen durch Triisocyanate ersetzt sein.

Geeignete zusätzliche Polymere sind weiterhin kationische Polymere. Dazu zählen z. B. Polymere mit der Bezeichnung Polyquaternium nach INCI, z. B. Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidocopolymere (Polyquaternium-7) und Chitosan. Geeignete kationische (quaternisierte) Polymere sind auch Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar®-Marken der Fa. Rhodia.

Geeignete zusätzliche Polymere sind auch amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw.

-Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).
Als zusätzliche Polymere geeignete neutrale Polymere sind z. B. Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex® Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol® Plus (BASF), oder Polyvinylpyrrolidon und deren Copolymere, insbesondere mit Vinylestern, wie Vinylacetat, z. B. Luviskol® VA 37 (BASF); Polyamide, z. B. auf Basis von Itaconsäure und aliphatischen Diaminen, wie sie z. B. in der DE-A-43 33 238 beschrieben sind.

Geeignete Polymere sind auch nichtionische, siloxanhaltige, wasserlösliche oder -dispergierbare Polymere, z. B. Polyethersiloxane, wie Tegopren® (Fa. Goldschmidt) oder Belsil® (Fa. Wacker).

Erfindungsgemäße Haarbehandlungsmittel enthalten vorzugsweise wenigstens ein erfindungsgemäßes Copolymer in einer Menge im Bereich von etwa 0,1 bis 30 Gew.-%, bevorzugt 0,5 bis 20 Gew.-%, bezogen auf das Gesamtgewicht des Mittels.

Erfindungsgemäß liegen die erfindungsgemäßen Haarbehandlungsmittel in Form eines Haarsprays, vor. Haarsprays umfassen dabei sowohl Aerosolsprays als auch Pumpsprays ohne Treibgas. Haarsprays umfassen vorzugsweise überwiegend oder ausschließlich wasserlösliche oder wasserdispergierbare Komponenten. Sind die in den erfindungsgemäßen Haarsprays eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Die erfindungsgemäßen haarkosmetischen Formulierungen enthalten in einer bevorzugten Ausführungsform
a) 0,05 bis 20 Gew.-% wenigstens eines Copolymers, wie zuvor definiert,
b) 20 bis 99,95 Gew.-% Wasser und/oder Alkohol,
c) 0 bis 50 Gew.-% wenigstens eines Treibgases,
d) 0 bis 5 Gew.-% wenigstens eines Emulgators,
e) 0 bis 3 Gew.-% wenigstens eines Verdickers, sowie
f) bis zu 25 Gew.-% weitere Bestandteile.

Unter Alkohol sind alle in der Kosmetik üblichen Alkohole zu verstehen, z. B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d. h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z. B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Eiweißhydrolysate, Stabilisatoren, pH-Wert-Regulatoren, Farbstoffe, Viskositätsregulierer, Gelbildner, Farbstoffe, Salze, Feuchthaltemittel, Rückfetter, Komplexbildner und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Copolymere eignen sich insbesondere als Festigungsmittel in Haarsprays (Aerosolsprays und Pumpsprays ohne Treibgas).

In einer bevorzugten Ausführungsform enthalten Spray-Zubereitungen
a) 0,1 bis 10 Gew.-% wenigstens eines Copolymers, wie zuvor definiert,
b) 20 bis 99,9 Gew.-% Wasser und/oder Alkohol,
c) 0 bis 70 Gew.-% wenigstens eines Treibmittel,
d) 0 bis 20 Gew.-% weitere Bestandteile.

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Die erfindungsgemäßen Copolymere können in kosmetischen Zubereitungen als Konditioniermittel eingesetzt werden.

Die Erfindung wird anhand der folgenden nicht einschränkenden Beispiele näher erläutert.

### Beispiele

Allgemeine Herstellungsvorschrift: Lösungspolymerisation in Ethanol/Wasser (2:1)

### Beispiel 15:

600 g einer 30 %igen Polymerlösung (TBA/ MAM/ NtBAM/ MAS = 63: 10: 10: 17 )

| | |
|---|---|
| Zulauf 1: | Monomerengemisch aus : |
| 113,4 g | tert.-Butylacrylat |
| 120 g | Methacrylsäureamid (15 %ig) |
| 18 g | N-tert.-Butylacrylamid |
| 30,6 g | Acrylsäure |
| 147 g | Ethanol |

| | |
|---|---|
| Zulauf 2: | Initiatorlösung aus: |
| 0,5 g Wako ® 50 [2,2'-Azobis(2-amindinopropan)dihydrochlorid] | |
| 21 g | Wasser |

| | |
|---|---|
| Zulauf 3: | Initiatorlösung aus: |
| 0,9 g | tert.-Butylperpivalat 75 %ig |
| 42 g | Ethanol |

| | |
|---|---|
| Zulauf 4: | Neutralisationsmittel: |
| 34 g | 2-Amino-2-methyl-propanol (AMP) |
| 97 g | Ethanol/ Wasser |

In einer Rührapparatur mit Rückflusskühler, Innenthermometer und vier separaten Zulaufvorrichtungen wurden 21 g von Zulauf 1, 1 g von Zulauf 2, 108 g Ethanol/Wasser (3,5 : 1) vorgelegt und die Mischung unter Rühren auf ca. 65 °C aufgeheizt. Nach dem Anpolymerisieren, erkennbar an einer leichten Viskositätserhöhung, wurde bei 67 °C der Rest von Zulauf 1 innerhalb von drei Stunden und der Rest von Zulauf 2 innerhalb von vier Stunden zugegeben. Die Reaktionslösung wurde noch ca. zwei Stunden bei 70 °C nachgerührt und anschließend der Zulauf 3 innerhalb von 30 Minuten bei 70 °C zudosiert. Nach der Zugabe wurde noch ca. zwei Stunden bei einer Temperatur von 80 °C nachpolymerisiert. Anschließend wurde zur Neutralisation der Zulauf 4 innerhalb von 10 Minuten zudosiert. Man erhielt eine ca. 30 %ige wässrig/ethanolische Lösung.

Analog wurden die Polymere Nr. 1 - 44 hergestellt.

**Tabelle 1**

| Bsp. Nr. | TBA | TMBA | MAM | NtBAM | ACMO | VP | DMAA | AS | MAS | NtBAE-MA | VI | DMAP -MAM | Amin/ N.G. [%] |
|---|---|---|---|---|---|---|---|---|---|---|---|---|---|
| 1 | 80 | - | 10 | - | - | - | - | 10 | - | - | - | - | AMP/95 |
| 2 | 75 | - | 15 | - | - | - | - | 10 | - | - | - | - | AMP/95 |
| 3 | 75 | - | 10 | - | - | - | - | 15 | - | - | - | - | AMP/90 |
| 4 | 73 | - | 10 | - | - | - | - | 17 | - | - | - | - | AMP/90 |
| 5 | 70 | - | 10 | - | - | - | - | 15 | 5 | - | - | - | AMP/85 |
| 6 | 70 | - | 10 | - | - | - | - | 20 | - | - | - | - | AMP/85 |
| 7 | 70 | - | 10 | - | - | - | - | 12 | 8 | - | - | - | AMP/85 |
| 8 | 70 | - | 10 | - | - | - | - | 10 | 10 | - | - | - | AMP/90 |
| 9 | 70 | - | 10 | - | - | - | - | 8 | 12 | - | - | - | AMP/95 |
| 10 | 65 | - | 15 | - | - | - | - | 10 | 10 | - | - | - | AMP/85 |
| 11 | 65 | - | - | 15 | - | - | - | 10 | 10 | - | - | - | AMP/85 |
| 12 | 65 | - | - | - | 15 | - | - | 10 | 10 | - | - | - | AMP/85 |
| 13 | 60 | - | - | 20 | - | - | - | 8 | 12 | - | - | - | AMP/95 |
| 14 | 60 | - | 10 | - | - | - | - | 8 | 22 | - | - | - | AMP/80 |
| 15 | 63 | - | 10 | 10 | - | - | - | 17 | - | - | - | - | AMP/90 |
| 16 | 60 | - | 15 | 10 | - | - | - | 15 | - | - | - | - | AMP/95 |
| 17 | 60 | - | 10 | 15 | - | - | - | 15 | - | - | - | - | AMP/95 |
| 18 | 55 | - | 10 | 20 | - | - | - | 15 | - | - | - | - | AMP/95 |
| 19 | 55 | - | 10 | 15 | - | - | - | 20 | - | - | - | - | AMP/85 |
| 20 | 50 | - | 13 | 20 | - | - | - | 17 | - | - | - | - | AMP/90 |
| 21 | 50 | - | 15 | - | - | 20 | - | 15 | - | - | - | - | AMP/80 |
| 22 | 50 | - | - | 15 | - | 20 | - | 15 | - | - | - | - | AMP/80 |
| 23 | 63 | 10 | 10 | - | - | - | - | 17 | - | - | - | - | AMP/95 |
| 24 | 63 | 10 | - | 10 | - | - | - | 17 | - | - | - | - | AMP/95 |
| 25 | 40 | 20 | - | 25 | - | | - | 15 | - | - | -- | - | AMP/95 |
| 26 | 55 | - | 20 | - | - | | - | 15 | 10 | - | - | - | AMP/85 |
| 27 | 55 | - | - | 20 | - | | - | 15 | 10 | - | - | - | AMP/85 |
| 28 | 55 | - | - | - | 20 | | - | 15 | 10 | - | - | - | AMP/85 |
| 29 | 55 | - | - | - | - | | 20 | 15 | 10 | - | - | - | AMP/85 |
| 30 | 70 | - | 5 | - | - | | - | 20 | - | 5 | - | - | AMP/85 |
| 31 | 65 | - | 10 | - | - | | - | 20 | - | 5 | - | - | AMP/85 |
| 32 | 65 | - | 15 | - | - | - | - | 15 | - | - | 5 | - | AMP/85 |
| 33 | 65 | - | 15 | - | - | | - | 17 | - | - | - | 3 | AMP/85 |
| 34 | 60 | - | 10 | - | - | | - | 10 | 12 | 8 | - | - | AMP/95 |
| 35 | 60 | - | 10 | - | - | - | - | 25 | - | - | 5 | - | AMP/90 |
| 36 | 60 | - | - | 12 | - | - | - | 22 | - | 6 | - | | AMP/90 |
| 37 | 55 | - | - | 20 | - | - | - | 8 | 12 | 5 | - | | AMP/95 |
| 38 | 30 | 30 | - | 12 | - | - | - | 22 | - | - | - | 6 | AMP/95 |
| 39 | 40 | - | 10 | - | - | 25 | - | 15 | - | 10 | - | | AMP/85 |

**Tabelle 2:**

| Beispiel Nr. | TBA | EMA | MAM | NtBAM | AS | MAS |
|---|---|---|---|---|---|---|
| 40 | 65 | - | - | 10 | 5 | 20 |
| 41 | 40 | 25 | 15 | - | 8 | 12 |
| 42 | 48 | 20 | - | 7 | 5 | 20 |
| 43 | 40 | 25 | - | 10 | 8 | 17 |
| 44 | 35 | 27 | - | 15 | 3 | 20 |

| | | | | | | |
|---|---|---|---|---|---|---|
| TBA: tert.-Butylacrylat TBMA: tert.-Butylmethacrylat EMA: Ethylmethacrylat MAM: Methacrylamid NtBAM: N-tert.-Butylacrylamid ACMO: Acryloylmorpholin VP: Vinylpyrrolidon DMAA: N,N-Dimethylacrylamid AS: Acrylsäure MAS: Methacrylsäure NtBAEMA: N-tert.-Butylaminoethylmethacrylat VI: Vinylimidazol DMAPMAM: Dimethylaminopropylmethacrylamid AMP: 2-Amino-2-methylpropanol N.G.: Neutralisationsgrad | | | | | | |

Anwendungstechnische Beispiele:
l) Verwendung in der Haarkosmetik:
1) VOC 80 Aerosol-Haarspray (Beispiele Nr. 1 - 39)

| | |
|---|---|
| Polymer 1-39 (30 %ige wässrig-ethanol. Lösung ) | 10,0 |
| Wasser | 14,6 |
| Dimethylether | 40,0 |
| Ethanol | 35,4 |

Weitere Zusatzstoffe:
Konservierungsmittel, lösliches ethoxiliertes Silikon, Parfüm, Entschäumer ...
2) VOC 80 Aerosol-Haarspray (Beispiele Nr. 40 - 78)

| | |
|---|---|
| Polymer 1-39 (30 %ige wässrig-ethanol. Lösung ) | 6,6 |
| Luvimer ® 100P | 1,0 (TBA/EA/MAS-Terpolymer, |
| | Fa. BASF) |
| Wasser | 15,5 |
| Dimethylether | 40,0 |
| Ethanol | 37,0 |

Weitere Zusatzstoffe:
Konservierungsmittel, lösliches ethoxiliertes Silikon, Parfüm, Entschäumer ...
3) VOC 55 Aerosol-Haarspray (Beispiele Nr. 79 - 122)

| | |
|---|---|
| Polymer 1-44 (30 %ige wässrig-ethanol. Lösung ) | 10,0 |
| Wasser | 39,6 |
| Dimethylether | 40,0 |
| Ethanol | 10,4 |

Weitere Zusatzstoffe:
Konservierungsmittel, lösliches ethoxiliertes Silikon, Parfüm, Entschäumer...
4) VOC 55 Aerosol-Haarspray (Beispiele Nr. 123 - 166)

| | |
|---|---|
| Polymer 1-44 (30 %ige wässrig-ethanol. Lösung) | 10,0 |
| Luviset ® PUR (30 %ige Lösung ) | 5,0 (Polyurethan, Fa. BASF) |
| Wasser | 35,1 |
| Dimethylether | 40,0 |
| Ethanol | 9,9 |

Weitere Zusatzstoffe:
Konservierungsmittel, lösliches ethoxiliertes Silikon, Parfüm, Entschäumer...
5) VOC 55 Pumpspray (Beispiele Nr. 167 - 210)

| | |
|---|---|
| Polymer 1-44 (30 %ige wässrig-ethanol. Lösung ) | 13,5 |
| Wasser | 37,8 |
| Ethanol | 48,7 |

Weitere Zusatzstoffe:
Konservierungsmittel, lösliches ethoxiliertes Silikon, Parfüm, Entschäumer...
6) Schaumfestiger (Beispiele Nr. 211 - 234)

| | |
|---|---|
| Polymer Nr. 6, 10, 12, 14, 21, 22, 26, 28, 29, 30, 31, 32, 33, 34, 35, 36, 37, 38, 39, 40, 41, 42, 43, 44 (30 %ige Lösung) | 10,0 |
| Cremophor ® A 25 | 0,2 (Ceteareth 25, Fa. BASF) |
| Comperlan ® KD | 0,1 (Coamide DEA, Fa. Henkel) |
| Wasser | 79.7 |
| Dimethylether | 10,0 |

Weitere Zusatzstoffe: Parfüm, Konservierungsmittel...
Herstellung : Einwiegen und unter Rühren lösen. Abfüllen und Treibgas zusetzen.
7) Schaumfestiger (Beispiele Nr. 235 - 258)

| | |
|---|---|
| Polymer Nr. 6, 10, 12, 14, 21, 22, 26, 28, 29, 30, 31, 32, 33, 34, 35, 36, 37, 38, 39, 40, 41, 42, 43, 44 (30 %ige Lösung) | 10,0 |
| Luviflex ® Soft (10 %ig wässrige Lösung, pH=7) | 15,0 (Acrylat-Copolymer, |
| | Fa. BASF) |
| Cremophor ® A 25 | 0,2 (Ceteareth 25, Fa. BASF) |
| Comperlan ® KD | 0,1 (Coamide DEA Fa. Henkel) |
| Wasser | 64,7 |
| Dimethylether | 10,0 |

Weitere Zusatzstoffe: Parfüm, Konservierungsmittel...
Herstellung : Einwiegen und unter Rühren lösen. Abfüllen und Treibgas zusetzen.
8) Haargel (Beispiele Nr. 259 - 282)

| | [ % ] |
|---|---|
| Phase 1: | |
| Polymer Nr. 6, 10, 12, 14, 21, 22, 26, 28, 29, 30, 31, 32, 33, 34, 35, 36, 37, 38, 39, 40, 41, 42, 43, 44 (30 %ige Lösung) | 10,0 |
| dest. Wasser | 39,0 |
| Aminomethylpropanol (38 %ige Lösung) | 1,0 |
| Weitere Zusatzstoffe: Konservierungsmittel, lösliches ethoxiliertes Silikon, Parfüm... | |
| Phase 2 : | |
| Acrylsäure/Beheneth-25-methacrylat-Copolymere (Aculyn® 28 von der Firma Rohm und Haas, 1 %ige wässrige Suspension ) | 50,0 |
| Herstellung : | Die Komponenten der Phase 1 und 2 werden getrennt eingewogen und homogenisiert. Phase 2 wird langsam in Phase 1 eingerührt. Es bildet sich ein klares, viskoses Gel. |

9) Haargel (Beispiele Nr. 283 - 306)

| | | [ % ] |
|---|---|---|
| Phase 1: | | |
| Polymer Nr. 6, 10, 12, 14, 21, 22, 26, 28, 29, 30, 31, 32, 33, 34, 35, 36, 37, 38, 39, 40, 41, 42, 43, 44 (30 %ige Lösung) | | 10,0 |
| dest. Wasser | | 40,0 |
| Weitere Zusatzstoffe: Konservierungsmittel, lösliches ethoxiliertes Silikon, Parfüm... | | |
| Phase 2 : | | |
| Hydroxyethylcellulose (Natrosol ® HR 250, Fa. Hercules 5 %ige wässrige Lösung) | | 50,0 |
| Herstellung : | Die Komponenten der Phase 1 und 2 werden getrennt eingewogen und homogenisiert. Phase 1 wird langsam in Phase 2 eingerührt. Es bildet sich ein klares, gießbares Gel. | |

II) Verwendung in der Hautkosmetik:
10) Standard O/W-Creme (Beispiele Nr. 307 - 322)

| Ölphase : | % | CTFA Name |
|---|---|---|
| Cremophor A6 | 3,5 | Ceteareth-6 (and) Stearyl Alkohol |
| Cremophor A25 | 3,5 | Ceteareth-25 |
| Glycerinmonostearat s.e. | 2,5 | Glyceryl Stearate |
| Paraffinöl | 7,5 | Paraffin Oil |
| Cetylalkohol | 2,5 | Cetyl Alkohol |
| Luvitol EHO | 3,2 | Cetearyl Octanoate |
| Vitamin-E-acetate | 1,0 | Tocopheryl Acetate |
| Nip-Nip | 0,1 | Methyl- und Propyl-4- |
| | | hydroxybenzoate (7:3) |
| Wasser-Phase: | | |
| | % | CTFA Name |

| | | | |
|---|---|---|---|
| Polymer Nr. 21, 30, 31, 32, 33, 34, 35, 36, 37, 38, 39, 40, 41, 42, 43, 44 | | 3,0 | |
| (20 %ige wässrige Lösung) Wasser | | 74,6 | |
| 1,2-Propylenglykol | | 1,5 | Propylene Glycol |
| Germall II | | 0,1 | Imidazolidinyl-Urea |
| Herstellung : | Die Komponenten werden eingewogen und die Öl-Phase und Wasser-Phase getrennt bei einer Temperatur von ca. 80 °C unter Rühren homogenisieren. Die Wasser-Phase wird langsam in die Öl-Phase eingerührt und die Mischung unter Rühren auf Raumtemperatur abgekühlt. | | |

## Patentansprüche

1. Haarspray enthaltend
A) wenigstens ein Copolymer, erhältlich durch radikalische Polymerisation eines Monomergemischs, enthaltend
a) tert.-Butylacrylat und/oder tert-Butylmethacrylat, wobei die Komponente a) tert.-Butylacrylat umfasst oder daraus besteht,
b) wenigstens eine α,β-ethylenisch ungesättigte amidgruppenhaltige Verbindung der allgemeinen Formel I 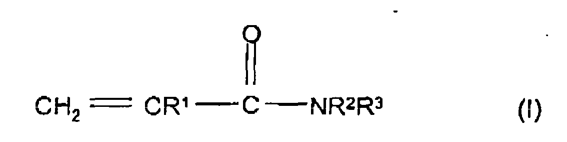 worin
R¹ für H oder C₁-C₄-Alkyl steht,
R² und R³ unabhängig voneinander für H oder C₁-C₄-Alkyl stehen oder R² und R³ gemeinsam mit dem Stickstoffatom, an das sie gebunden sind, auch für einen 4- bis 7-gliedrigen Heterocyclus stehen können,
mit der Maßgabe, dass die Summe der Kohlenstoffatome der Reste R¹, R² und R³ höchstens 4 beträgt, und
c) Acrylsäure, und
B) wenigstens einen kosmetisch oder pharmazeutisch akzeptablen Träger.

2. Haarspray nach Anspruch 1, wobei die Komponente b) ausgewählt ist unter Acrylsäureamid, Methacrylsäureamid,
N-Methyl(meth)acrylamid, N-Ethyl(meth)acrylamid, N-Propyl(meth)acrylamid,
N-(n-Butyl)acrylamid, N-(sek.-Butyl)acrylamid,
N-(tert.-Butyl)acrylamid, N,N-Dimethyl(meth)acrylamid,
N,N-Diethylacrylamid, N-Acryloylmorpholin, N-Acryloylpiperazin,
N-(Meth)acryloylpyrrolidin und Mischungen davon.

3. Haarspray nach einem der vorhergehenden Ansprüche, wobei die Komponente b) Methacrylsäureamid und/oder N-(tert.-Butyl)acrylamid umfasst oder aus Methacrylsäureamid und/oder N-(tert.-Butyl)acrylamid besteht.

4. Haarspray nach einem der vorhergehenden Ansprüche, das zusätzlich als Komponente d) Methacrylsäure einpolymerisiert enthält.

5. Haarspray nach einem der vorhergehenden Ansprüche, das zusätzlich als Komponente e) wenigstens eine Verbindung mit einer radikalisch polymerisierbaren, α,β-ethylenisch ungesättigten Doppelbindung und wenigstens einer kationogenen und/oder kationischen Gruppe pro Molekül, einpolymerisiert enthält.

6. Haarspray nach Anspruch 5, wobei die Komponente e) ausgewählt ist unter Estern α,β-ethylenisch ungesättigter Mono- und Dicarbonsäuren mit Aminoalkoholen, welche am Aminstickstoff mono- oder dialkyliert sein können, Amiden α,β-ethylenisch ungesättigter Mono- und Dicarbonsäuren mit Diaminen, welche mindestens eine primäre oder sekundäre Aminogruppe aufweisen, N,N-Diallylamin, N,N-Diallyl-N-alkylaminen und deren Derivaten, vinyl- und allylsubstituierten Stickstoffheterocyclen, vinyl- und allylsubstituierten heteroaromatischen Verbindungen und Mischungen davon.

7. Haarspray nach Anspruch 6, wobei die Komponente e) wenigstens eine Verbindung umfasst, die ausgewählt ist unter N-(tert.-Butylamino)ethyl(meth)acrylat, N,N-Dimethylaminoethyl(meth)acrylat,
N-[3-(dimethylamino)propyl](meth)acrylamid, Vinylimidazol und Mischungen davon.

8. Haarspray nach einem der vorhergehenden Ansprüche, das zusätzlich wenigstens ein weiteres Monomer f) einpolymerisiert enthält, das ausgewählt ist unter von Komponente a) verschiedenen Estern α,β-ethylenisch ungesättigter Mono- und Dicarbonsäuren mit C₁-C₃₀-Alkanolen und C₁-C₃₀-Alkandiolen, Amiden
α,β-ethylenisch ungesättigter Mono- und Dicarbonsäuren mit
C₂-C₃₀-Aminoalkoholen, die eine primäre oder sekundäre Aminogruppe aufweisen, N-Vinyllactamen, N-Vinylamiden gesättigter Monocarbonsäuren, von Komponente b) verschiedenen primären Amiden α,β-ethylenisch ungesättigter Monocarbonsäuren und deren N-Alkyl- und N,N-Dialkylderivaten, Estern von Vinylalkohol und Allylalkohol mit C₁-C₃₀-Monocarbonsäuren, Vinylethern, Vinylaromaten, Vinylhalogeniden, Vinylidenhalogeniden, C₁-C₈-Monoolefinen, nicht aromatischen Kohlenwasserstoffen mit mindestens zwei konjugierten Doppelbindungen und Mischungen davon.

9. Haarspray nach Anspruch 8, wobei die Komponente f) ausgewählt ist unter C₁-C₃-Alkylmethacrylaten, Hydroxy-C₁-C₃-alkylmethacrylaten und Mischungen davon.

10. Haarspray nach Anspruch 9, wobei die Komponente f) Ethylmethacrylat umfasst oder daraus besteht.

11. Haarspray nach einem der vorhergehenden Ansprüche, das
- 30 bis 90 Gew.-%, besonders bevorzugt 40 bis 85 Gew.-%, wenigstens einer Verbindung a),
- 3 bis 50 Gew.-%, besonders bevorzugt 5 bis 40 Gew.-%, insbesondere 10 bis 35 Gew.-%, wenigstens einer Verbindung b).
- 5 bis 40 Gew.-%, besonders bevorzugt 7 bis 35 Gew.-%, Insbesondere 10 bis 30 Gew.-%, Acrylsäure c),
- 0 bis 25 Gew.-%, besonders bevorzugt 1 bis 20 Gew.-%, insbesondere 5 bis 15 Gew.-%, Methacrylsäure d).
- 0 bis 25 Gew.-%, besonders bevorzugt 1 bis 20 Gew.-%, insbesondere 2 bis 10 Gew.-%, wenigstens einer Verbindung e),
- 0 bis 25 Gew.-%, besonders bevorzugt 0,1 bis 20 Gew.-%, insbesondere 5 bis 15 Gew.-%, wenigstens einer Verbindung f),
- 0 bis 5 Gew.-%, besonders bevorzugt 0,01 bis 3 Gew.-%, insbesondere 0,1 bis 2 Gew.-%, wenigstens eines Vernetzers g),
einpolymerisiert enthält.

12. Haarspray nach einem der Ansprüche 1 bis 11, das aus Wiederholungseinheiten von
- tert.-Butylacrylat,
- Methacrylsäureamid und/oder N-(tert.-Butyl)acrylamid,
- Acrylsäure
besteht.

13. Haarspray nach einem der Ansprüche 1 bis 11, das aus Wiederholungseinheiten von
- tert.-Butylacrylat,
- Methacrylsäureamid und/oder N-(tert.-Butyl)acrylamid,
- Acrylsäure und
- Methacrylsäure
besteht.

14. Haarspray nach einem der Ansprüche 1 bis 11, das aus Wiederholungseinheiten von
- tert.-Butylacrylat,
- N-Acryloylmorpholin oder N,N-Dimethylacrylamid und
- Acrylsäure
besteht.

15. Haarspray nach einem der Ansprüche 1 bis 11, das aus Wiederholungseinheiten von
- tert.-Butylacrylat,
- Methacrylsäureamid und/oder N-(tert.-Butyl)acrylamid,
- N-Vinylpyrrolidon und
- Acrylsäure
besteht.

16. Haarspray nach einem der Ansprüche 1 bis 11, das aus Wiederholungseinheiten von
- tert.-Butylacrylat.
- Methacrylsäureamid und/oder N-(tert.-Butyl)acrylamid,
- N-Vinylpyrrolidon;
- Acrylsäure und
- Methacrylsäure
besteht.

17. Haarspray nach einem der Ansprüche 1 bis 11, das aus Wiederholungseinheiten von
- tert.-Butylacrylat,
- tert.-Butylmethacrylat,
- Methacrylsäureamid und/oder N-(tert.-Butyl)acrylamid, und
- Acrylsäure
besteht.

18. Haarspray nach einem der Ansprüche 1 bis 11, das aus Wiederholungseinheiten von
- tert.-Butylacrylat,
- tert.-Butylmethacrylat,
- Methacrylsäureamid und/oder N-(tert.-Butyl)acrylamid,
- Acrylsäure und
- Methacrylsäure
besteht.

19. Haarspray nach einem der Ansprüche 1 bis 11, das aus Wiederholungseinheiten von
- tert.-Butylacrylat und/oder tert.-Butylmethacrylat,
- Methacrylsäureamid und/oder N-(tert.-Butyl)acrylamid,
- Acrylsäure und
- N-(tert.-Butyl)aminoethyl(meth)acrylat oder N-[3-(dimethylamino)propyl]methacrylamid oder N-Vinylimidazol
besteht.

20. Haarspray nach einem der Ansprüche 1 bis 11, das aus Wiederholungseinheiten von
- tert.-Butylacrylat und/oder tert.-Butylmethacrylat,
- Methacrylsäureamid und/oder N-(tert.-Butyl)acrylamld,
- Acrylsäure,
- Methacrylsäure und
- N-(tert.-Butyl)aminoethyl(meth)acrylat oder N-[3-(dimethylamino)propyl]methacrylamid oder N-Vinylimidazol
besteht.

21. Haarspray nach einem der Ansprüche 1 bis 11, das
a) tert.-Butylacrylat und/oder tert.-Butylmethacrylat,
b) Methacrylsäureamid und/oder N-(tert.-Butyl)acrylamid,
c) Acrylsäure und
f) wenigstens eine Verbindung, die ausgewählt ist unter C₁-C₃-Alkylmethacrylaten, Hydroxy-C₁-C₃-alkylmethacrylaten und Mischungen davon,
einpolymerisiert enthält, mit der Maßgabe, dass der Gewichtsmengenanteil der Komponente a) gleich ist wie oder größer ist als der Gewichtsmengenanteil der Komponente f).

22. Haarspray nach Anspruch 21, das aus Wiederholungseinheiten von
- tert.-Butylacrylat,
- Methacrylsäureamid und/oder N-(tert.-Butyl)acrylamid,
- Acrylsäure Methacrylsäure und
- Ethylmethacrylat
besteht.

23. Haarspray nach einem der Ansprüche 1 bis 11, das jeweils bezogen auf das Gesamtgewicht der zur Polymerisation eingesetzten Monomere,
- 40 bis 80 Gew.-%, bevorzugt 45 bis 75 Gew.-%, tert.-Butylacrylat und Ethylmethacrylat, mit der Maßgabe, dass der Gewichtsanteil an tert.-Butylmethacrylat gleich ist wie oder größer ist als der Gewichtsmengenanteil von Ethylmethacrylat,
- 5 bis 30 Gew.-%, bevorzugt 7 bis 25 Gew.-%, Methacrylsäureamid und/oder N-(tert.-Butyl)acrylamid,
- 5 bis 30 Gew.-%, bevorzugt 5 bis 15 Gew.-%, Acrylsäure und
- 0 bis 25 Gew.-%, bevorzugt 5 bis 20 Gew.-%, Methacrylsäure
einpolymerisiert enthält.

24. Haarspray nach einem der vorhergehenden Ansprüche, wobei die Komponente B) ausgewählt ist unter
i) Wasser,
ii) wassermischbaren organischen Lösungsmitteln, vorzugsweise C₂-C₄-Alkanolen, insbesondere Ethanol,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen
und Mischungen davon.

25. Haarspray nach einem der vorhergehenden Ansprüche, enthaltend wenigstens einen von den Komponenten A) und B) verschiedenen Zusatzstoff, der ausgewählt ist unter kosmetisch aktiven Wirkstoffen, Emulgatoren, Tensiden, Konservierungsmitteln, Parfümölen, Verdickem, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Färbemitteln, Tönungsmitteln, Bräunungsmittein, Farbstoffen, Pigmenten, Konsistenzgebern. Feuchthaltemitteln, Rückfettern, Collagen, Eiweisshydrolysaten, Lipiden, Antioxidantien, Entschäumern, Antistatika, Emollienzien und Weichmachern.

26. Verwendung eines Copolymers, wie in einem der Ansprüche 1 bis 23 definiert, in Haarbehandlungsmitteln
als Festiger und/oder als Conditioner, wobei das Mittel in Form eines Haarsprays vorliegt.

## Claims

1. A hair spray comprising
A) at least one copolymer obtainable by free radical polymerization of a monomer mixture comprising
a) tert-butyl acrylate and/or tert-butyl methacrylate, wherein the component a) comprises tert-butyl acrylate or consists thereof,
b) at least one α,β-ethylenically unsaturated amido-containing compound of the formula I 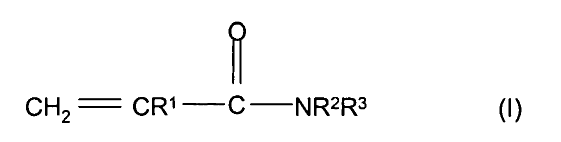 where
R¹ is H or C₁-C₄-alkyl,
R² and R³, independently of one another, are H or C₁-C₄-alkyl or R² and R³ together with the nitrogen atom to which they are bonded may also be a 4- to 7-membered heterocycle, with the proviso that the sum of the carbon atoms of the radicals R¹, R² and R³ is not more than 4, and
c) acrylic acid, and
B) at least one cosmetically or pharmaceutically acceptable carrier.

2. The hair spray according to claim 1, wherein the component b) is selected from acrylamide, methacrylamide, N-methyl(meth)acrylamide, N-ethyl-(meth)acrylamide, N-propyl(meth)acrylamide, N-(n-butyl)acrylamide, N-(sec-butyl)acrylamide, N-(tert-butyl)acrylamide, N,N-dimethyl(meth)acrylamide, N,N-diethylacrylamide, N-acryloylmorpholine, N-acryloylpiperazine, N-(meth)acryloylpyrrolidine and mixtures thereof.

3. The hair spray according to any of the preceding claims, wherein the component b) comprises methacrylamide and/or N-(tert-butyl)acrylamide or consists of methacrylamide and/or N-(tert-butyl)acrylamide.

4. The hair spray according to any of the preceding claims, which additionally comprises methacrylic acid incorporated in the form of polymerized units as component d).

5. The hair spray according to any of the preceding claims, which additionally comprises, as component e), incorporated in the form of polymerized units, at least one compound having an α,β-ethylenically unsaturated double bond capable of free radical polymerization and at least one cationogenic and/or cationic group per molecule.

6. The hair spray according to claim 5, wherein the component e) is selected from esters of α,β-ethylenically unsaturated mono- and dicarboxylic acids with amino alcohols which may be mono- or dialkylated on the amine nitrogen, amides of α,β-ethylenically unsaturated mono- and dicarboxylic acids with diamines which have at least one primary or secondary amino group, N,N-diallylamine, N,N-diallyl-N-alkylamines and derivatives thereof, vinyl- and allyl-substituted nitrogen heterocycles, vinyl- and allyl-substituted heteroaromatic compounds and mixtures thereof.

7. The hair spray according to claim 6, wherein the component e) comprises at least one compound which is selected from N-(tert-butylamino)ethyl (meth)acrylate, N,N-dimethylaminoethyl (meth) acrylate, N-[3-(dimethylamino)propyl](meth)acrylamide, vinylimidazole and mixtures thereof.

8. The hair spray according to any of the preceding claims, which additionally comprises, incorporated in the form of polymerized units, at least one further monomer f) which is selected from esters of α,β-ethylenically unsaturated mono- and dicarboxylic acids with C₁-C₃₀-alkanols and C₁-C₃₀-alkanediols, which esters differ from component a), amides of α,β-ethylenically unsaturated mono- and dicarboxylic acids with C₂-C₃₀-amino alcohols which have a primary or secondary amino group, N-vinyllactams, N-vinylamides of saturated monocarboxylic acids, primary amides of α,β-ethylenically unsaturated monocarboxylic acids and N-alkyl and N,N-dialkyl derivatives thereof, which primary amides differ from component b), esters of vinyl alcohol and allyl alcohol with C₁-C₃₀-monocarboxylic acids, vinyl ethers, vinylaromatics, vinyl halides, vinylidene halides, C₁-C₈-monoolefins, nonaromatic hydrocarbons having at least two conjugated double bonds and mixtures thereof.

9. The hair spray according to claim 8, the component f) being selected from C₁-C₃-alkyl methacrylates, hydroxy-C₁-C₃-alkyl methacrylates and mixtures thereof.

10. The hair spray according to claim 9, the component f) comprising or consisting of ethyl methacrylate.

11. The hair spray according to any of the preceding claims, which comprises, incorporated in the form of polymerized units,
- from 30 to 90, particularly preferably from 40 to 85, % by weight of at least one compound a),
- from 3 to 50, particularly preferably from 5 to 40, in particular from 10 to 35, % by weight of at least one compound b),
- from 5 to 40, particularly preferably from 7 to 35, in particular from 10 to 30, % by weight of acrylic acid c),
- from 0 to 25, particularly preferably from 1 to 20, in particular from 5 to 15, % by weight of methacrylic acid d),
- from 0 to 25, particularly preferably from 1 to 20, in particular from 2 to 10, % by weight of at least one compound e),
- from 0 to 25, particularly preferably from 0.1 to 20, in particular from 5 to 15, % by weight of at least one compound f),
- from 0 to 5, particularly preferably from 0.01 to 3, in particular from 0.1 to 2, % by weight of at least one crosslinking agent g).

12. The hair spray according to any of claims 1 to 11, which consists of repeating units of
- tert-butyl acrylate,
- methacrylamide and/or N-(tert-butyl)acrylamide,
- acrylic acid.

13. The hair spray according to any of claims 1 to 11, which consists of repeating units of
- tert-butyl acrylate,
- methacrylamide and/or N-(tert-butyl)acrylamide,
- acrylic acid and
- methacrylic acid.

14. The hair spray according to any of claims 1 to 11, which consists of repeating units of
- tert-butyl acrylate,
- N-acryloylmorpholine or N,N-dimethylacrylamide and
- acrylic acid.

15. The hair spray according to any of claims 1 to 11, which consists of repeating units of
- tert-butyl acrylate,
- methacrylamide and/or N-(tert-butyl)acrylamide,
- N-vinylpyrrolidone and
- acrylic acid.

16. The hair spray according to any of claims 1 to 11, which consists of repeating units of
- tert-butyl acrylate,
- methacrylamide and/or N-(tert-butyl)acrylamide,
- N-vinylpyrrolidone,
- acrylic acid and
- methacrylic acid.

17. The hair spray according to any of claims 1 to 11, which consists of repeating units of
- tert-butyl acrylate,
- tert-butyl methacrylate,
- methacrylamide and/or N-(tert-butyl)acrylamide and
- acrylic acid.

18. The hair spray according to any of claims 1 to 11, which consists of repeating units of
- tert-butyl acrylate,
- tert-butyl methacrylate,
- methacrylamide and/or N-(tert-butyl)acrylamide,
- acrylic acid and
- methacrylic acid.

19. The hair spray according to any of claims 1 to 11, which consists of repeating units of
- tert-butyl acrylate and/or tert-butyl methacrylate,
- methacrylamide and/or N-(tert-butyl)acrylamide,
- acrylic acid and
- N-(tert-butyl)aminoethyl (meth)acrylate or N-[3-(dimethylamino)propyl]methacrylamide or N-vinylimidazole.

20. The hair spray according to any of claims 1 to 11, which consists of repeating units of
- tert-butyl acrylate and/or tert-butyl methacrylate,
- methacrylamide and/or N-(tert-butyl)acrylamide,
- acrylic acid,
- methacrylic acid and
- N-(tert-butyl)aminoethyl (meth)acrylate or N-[3-(dimethylamino)propyl]methacrylamide or N-vinylimidazole.

21. The hair spray according to any of claims 1 to 11, which comprises, incorporated in the form of polymerized units,
a) tert-butyl acrylate and/or tert-butyl methacrylate,
b) methacrylamide and/or N-(tert-butyl)acrylamide,
c) acrylic acid and
f) at least one compound which is selected from C₁-C₃-alkyl methacrylates, hydroxy-C₁-C₃-alkyl methacrylates and mixtures thereof,
with the proviso that the proportion by weight of component a) is equal to or greater than the proportion by weight of component f).

22. The hair spray according to claim 21, which consists of repeating units of
- tert-butyl acrylate,
- methacrylamide and/or N-(tert-butyl)acrylamide,
- acrylic acid
- methacrylic acid and
- ethyl methacrylate.

23. The hair spray according to any of claims 1 to 11, which comprises, based in each case on the total weight of the monomers used for the polymerization,
- from 40 to 80% by weight, preferably from 45 to 75% by weight, of tert-butyl acrylate and ethyl methacrylate, with the proviso that the proportion by weight of tert-butyl methacrylate is equal to or greater than the proportion by weight of ethyl methacrylate,
- from 5 to 30% by weight, preferably from 7 to 25% by weight, of methacrylamide and/or N-(tert-butyl)acrylamide,
- from 5 to 30% by weight, preferably from 5 to 15% by weight, of acrylic acid and
- from 0 to 25% by weight, preferably from 5 to 20% by weight, of methacrylic acid,
incorporated in the form of polymerized units.

24. The hair spray according to any of the preceding claims, wherein the component B) is selected from
i) water,
ii) water-miscible organic solvents, preferably C₂-C₄-alkanols, in particular ethanol,
iii) oils, fats, waxes,
iv) esters of C₆-C₃₀-monocarboxylic acids with monohydric, dihydric or trihydric alcohols, which esters differ from iii),
v) saturated acyclic and cyclic hydrocarbons,
vi) fatty acids,
vii) fatty alcohols,
viii) propellants
and mixtures thereof.

25. The hair spray according to any of the preceding claims, comprising at least one additive which differs from the components A) and B) and is selected from cosmetically active substances, emulsifiers, surfactants, preservatives, perfume oils, thickeners, hair polymers, hair and skin conditioners, graft polymers, water-soluble or dispersible silicone-containing polymers, light stabilizers, bleaches, gel formers, care compositions, colorants, tinting compositions, tanning compositions, dyes, pigments, consistency agents, humidifiers, refatting agents, collagen, protein hydrolysis products, lipids, antioxidants, antifoams, antistatic agents, emollients and softeners.

26. The use of a copolymer, as defined in any of claims 1 to 23, in hair treatment compositions as setting compositions and/or as conditioners, wherein the composition is present in the form of a hair spray.

## Revendications

1. Laque pour cheveux, contenant :
A) au moins un copolymère, que l'on peut obtenir par polymérisation par voie radicalaire d'un mélange monomère, contenant :
a) de l'acrylate de tert-butyle et/ou du méthacrylate de tert-butyle, le composant a) comprenant de l'acrylate de tert-butyle ou en étant constitué,
b) au moins un composé α,β-éthyléniquement insaturé contenant des groupes amide de formule générale I : 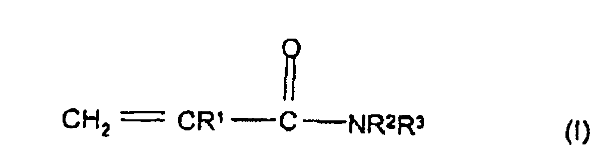 dans laquelle
R¹ représente H ou de l'alkyle en C₁-C₄,
R² et R³ représentent indépendamment l'un de l'autre H ou de l'alkyle en C₁-C₄, ou R² et R³ peuvent représenter également, en commun avec l'atome d'azote auquel ils sont liés, un hétérocycle carré à heptagonal, sous réserve que la somme des atomes de carbone des radicaux R¹, R² et R³ soit d'au plus 4, et
c)de l'acide acrylique, et
B) au moins un support cosmétiquement ou pharmaceutiquement acceptable.

2. Laque pour cheveux selon la revendication 1, dans laquelle le composant b) est choisi parmi l'amide d'acide acrylique, l'amide d'acide méthacrylique, le (méth)acrylamide de N-méthyle, le (méth)acrylamide de N-éthyle, le (méth)acrylamide de N-propyle, l'acrylamide de N-(n-butyle), l'acrylamide de N-(sec-butyle), l'acrylamide de N-(tert-butyle), le (méth)acrylamide de N,N-diméthyle, l'acrylamide de N,N-diéthyle, la morpholine de N-acryloyle, la pipérazine de N-acryloyle, la pyrrolidine de N-(méth)acryloyle et leurs mélanges.

3. Laque pour cheveux selon l'une quelconque des revendications précédentes, dans laquelle le composant b) comprend de l'amide d'acide méthacrylique et/ou de l'acrylamide de N-(tert-butyle) ou est constitué d'amide d'acide méthacrylique et/ou d'acrylamide de N-(tert-butyle).

4. Laque pour cheveux selon l'une quelconque des revendications précédentes, qui contient à l'état polymérisé de l'acide méthacrylique de manière supplémentaire, en tant que composant d).

5. Laque pour cheveux selon l'une quelconque des revendications précédentes, qui contient à l'état polymérisé au moins un composé ayant une double liaison α,β-éthyléniquement insaturée, pouvant être polymérisée par voie radicalaire et au moins un groupe cationogène et/ou cationique par molécule, de manière supplémentaire en tant que composant e).

6. Laque pour cheveux selon la revendication 5, dans laquelle le composant e) est choisi parmi des esters des acides monocarboxyliques et dicarboxyliques α,β-éthyléniquement insaturés avec des aminoalcools, qui peuvent être monoalkylés ou dialkylés au niveau de l'azote aminé, des amides d'acides monocarboxyliques et dicarboxyliques α,β-éthyléniquement insaturés avec des diamines, qui présentent au moins un groupe amino primaire ou secondaire, de la N,N-diallylamine, des N,N-diallyl-N-alkylamines et leurs dérivés, des hétérocycles azotés substitués par du vinyle et des hétérocycles azotés substitués par de l'allyle, des composés hétéroaromatiques substitués par du vinyle et des composés hétéroaromatiques substitués par de l'allyle et leurs mélanges.

7. Laque pour cheveux selon la revendication 6, dans laquelle le composant e) comprend au moins un composé choisi parmi du (méth)acrylate de N-(tert-butylamino)éthyle, du (méth)acrylate de N,N-diméthylaminoéthyle, du (méth)acrylamide de N-[3-(diméthylamino)propyle], du vinylimidazole et leurs mélanges.

8. Laque pour cheveux selon l'une quelconque des revendications précédentes, qui contient à l'état polymérisé de manière supplémentaire au moins un autre monomère f) choisi parmi des esters, différents du composant a), d'acides monocarboxyliques et dicarboxyliques α,β-éthyléniquement insaturés avec des alcanols en C₁-C₃₀ et des alcanediols en C₁-C₃₀, des amides d'acides monocarboxyliques et dicarboxyliques α,β-éthyléniquement insaturés avec des aminoalcools en C₂-C₃₀, qui présentent un groupe amino primaire ou secondaire, des N-vinyllactames, des N-vinylamides d'acides monocarboxyliques saturés, des amides primaires, différents du composant b), d'acides monocarboxyliques α,β-éthyléniquement insaturés et leurs dérivés N-alkyliques et N,N-dialkyliques, des esters d'alcool vinylique et d'alcool allylique avec des acides monocarboxyliques en C₁-C₃₀, des éthers de vinyle, des substances vinylaromatiques, des halogénures de vinyle, des halogénures de vinylidène, des mono-oléfines en C₁-C₈, des hydrocarbures non aromatiques ayant au moins deux doubles liaisons conjuguées et leurs mélanges.

9. Laque pour cheveux selon la revendication 8, dans laquelle le composant f) est choisi parmi des méthacrylates d'alkyle en C₁-C₃, des méthacrylates d'hydroxyalkyle en C₁-C₃ et leurs mélanges.

10. Laque pour cheveux selon la revendication 9, dans laquelle le composant f) comprend du méthacrylate d'éthyle ou en est constitué.

11. Laque pour cheveux selon l'une quelconque des revendications précédentes, qui contient à l'état polymérisé :
- 30 à 90 % en poids, mieux encore 40 à 85 % en poids, d'au moins un composé a),
- 3 à 50 % en poids, mieux encore 5 à 40 % en poids, en particulier 10 à 35 % en poids, d'au moins un composé b),
- 5 à 40 % en poids, mieux encore 7 à 35 % en poids, en particulier 10 à 30 % en poids, d'acide acrylique c),
- 0 à 25 % en poids, mieux encore 1 à 20 % en poids, en particulier 5 à 15 % en poids, d'acide méthacrylique d),
- 0 à 25 % en poids, mieux encore 1 à 20 % en poids, en particulier 2 à 10 % en poids, d'au moins un composé e),
- 0 à 25 % en poids, mieux encore 0,1 à 20 % en poids, en particulier 5 à 15 % en poids, d'au moins un composé f),
- 0 à 5 % en poids, mieux encore 0,01 à 3 % en poids, en particulier 0,1 à 2 % en poids, d'au moins un agent de réticulation g).

12. Laque pour cheveux selon l'une quelconque des revendications 1 à 11, qui est constituée d'unités de répétition de :
- acrylate de tert-butyle,
- amide d'acide méthacrylique et/ou acrylamide de N-(tert-butyle),
- acide acrylique.

13. Laque pour cheveux selon l'une quelconque des revendications 1 à 11, qui est constituée d'unités de répétition de :
- acrylate de tert-butyle,
- amide d'acide méthacrylique et/ou acrylamide de N-(tert-butyle),
- acide acrylique et
- acide méthacrylique.

14. Laque pour cheveux selon l'une quelconque des revendications 1 à 11, qui est constituée d'unités de répétition de :
- acrylate de tert-butyle,
- morpholine de N-acryloyle ou acrylamide de N,N-diméthyle et
- acide acrylique.

15. Laque pour cheveux selon l'une quelconque des revendications 1 à 11, qui est constituée d'unités de répétition de :
- acrylate de tert-butyle,
- amide d'acide méthacrylique et/ou acrylamide de N-(tert-butyle),
- N-vinylpyrrolidone et
- acide acrylique.

16. Laque pour cheveux selon l'une quelconque des revendications 1 à 11, qui est constituée d'unités de répétition de :
- acrylate de tert-butyle,
- amide d'acide méthacrylique et/ou acrylamide de N-(tert-butyle),
- N-vinylpyrrolidone ;
- acide acrylique et
- acide méthacrylique.

17. Laque pour cheveux selon l'une quelconque des revendications 1 à 11, qui est constituée d'unités de répétition de :
- acrylate de tert-butyle,
- méthacrylate de tert-butyle,
- amide d'acide méthacrylique et/ou acrylamide de N-(tert-butyle) et
- acide acrylique.

18. Laque pour cheveux selon l'une quelconque des revendications 1 à 11, qui est constituée d'unités de répétition de :
- acrylate de tert-butyle,
- méthacrylate de tert-butyle,
- amide d'acide méthacrylique et/ou acrylamide de N-(tert-butyle),
- acide acrylique et
- acide méthacrylique.

19. Laque pour cheveux selon l'une quelconque des revendications 1 à 11, qui est constituée d'unités de répétition de :
- acrylate de tert-butyle et/ou méthacrylate de tert-butyle,
- amide d'acide méthacrylique et/ou acrylamide de N-(tert-butyle),
- acide acrylique et
- (méth)acrylate de N-(tert-butyl)aminoéthyle ou méthacrylamide de N-[3-(diméthylamino)propyle] ou N-vinylimidazole.

20. Laque pour cheveux selon l'une quelconque des revendications 1 à 11, qui est constituée d'unités de répétition de :
- acrylate de tert-butyle et/ou méthacrylate de tert-butyle,
- amide d'acide méthacrylique et/ou acrylamide de N-(tert-butyle),
- acide acrylique,
- acide méthacrylique et
- (méth)acrylate de N-(tert-butyl)aminoéthyle ou méthacrylamide de N-[3-(diméthylamino)propyle] ou N-vinylimidazole.

21. Laque pour cheveux selon l'une quelconque des revendications 1 à 11, qui contient à l'état polymérisé :
a) de l'acrylate de tert-butyle et/ou du méthacrylate de tert-butyle,
b) de l'amide d'acide méthacrylique et/ou de l'acrylamide de N-(tert-butyle),
c) de l'acide acrylique et
f) au moins un composé, qui est choisi parmi des méthacrylates d'alkyle en C₁-C₃, des méthacrylates d'hydroxyalkyle en C₁-C₃ et leurs mélanges,
sous réserve que la part en poids du composant a) soit identique ou supérieure à la part en poids du composant f).

22. Laque pour cheveux selon la revendication 21, qui est constituée d'unités de répétition de :
- acrylate de tert-butyle,
- amide d'acide méthacrylique et/ou acrylamide de N-(tert-butyle),
- acide acrylique,
- acide méthacrylique et
- méthacrylate d'éthyle.

23. Laque pour cheveux selon l'une quelconque des revendications 1 à 11, qui contient à l'état polymérisé, respectivement par rapport au poids total des monomères utilisés pour la polymérisation :
- 40 à 80 % en poids, de préférence 45 à 75 % en poids, d'acrylate de tert-butyle et de méthacrylate d'éthyle, sous réserve que la part en poids de méthacrylate de tert-butyle soit identique à ou supérieure à la part en poids du méthacrylate d'éthyle,
- 5 à 30 % en poids, de préférence 7 à 25 % en poids, d'amide d'acide méthacrylique et/ou d'acrylamide de N-(tert-butyle),
- 5 à 30 % en poids, de préférence 5 à 15 % en poids, d'acide acrylique et
- 0 à 25 % en poids, de préférence 5 à 20 % en poids, d'acide méthacrylique.

24. Laque pour cheveux selon l'une quelconque des revendications précédentes, dans laquelle le composant B) est choisi parmi :
i) de l'eau,
ii) des solvants organiques miscibles dans l'eau, de préférence des alcanols en C₂-C₄, en particulier de l'éthanol,
iii) des huiles, des graisses, des cires,
iv) des esters, différents de iii), d'acides monocarboxyliques en C₆-C₃₀ avec des alcools monovalents, bivalents ou trivalents,
v) des hydrocarbures acycliques et cycliques saturés,
vi) des acides gras,
vii)des alcools gras,
viii) des gaz propulseurs
et leurs mélanges.

25. Laque pour cheveux selon l'une quelconque des revendications précédentes, contenant au moins un additif, différent des composants A) et B), choisi parmi des substances actives cosmétiquement actives, des émulsifiants, des tensioactifs, des conservateurs, des huiles parfumées, des épaississants, des polymères pour cheveux, des conditionneurs pour les cheveux et la peau, des polymères greffés, des polymères contenant du silicone, solubles ou dispersibles dans l'eau, des agents de protection contre la lumière, des agents de blanchiment, des formateurs de gel, des produits de soin, des colorants, des produits de coloration, des produits de bronzage, des colorants, des pigments, des ajusteurs de consistance, des produits hydratants, des regraissants, du collagène, des hydrolysats de protéine, des lipides, des antioxydants, des agents antimousse, des produits antistatiques, des émollients et des adoucissants.

26. Utilisation d'un copolymère, tel que défini dans l'une quelconque des revendications 1 à 23, en tant que fixateur et/ou en tant que conditionneur dans des produits de traitement capillaire, le produit se présentant sous la forme d'une laque pour cheveux.
